# EUROPEAN PATENT APPLICATION

(11) **EP 4 456 079 A1**
(43) Date of publication of application: **30.10.2024**
(21) Application number: 24168169.1
(22) Date of filing: 03.04.2024
(51) Int. Cl.: G16H 10/60, G16H 15/00, G16H 40/63, G16H 40/67, G16H 50/20

(54) **SYSTEMS AND METHODS FOR GLOBAL PATIENT MEDICAL DEVICE DATA PROCESSING**

(30) Priority: 26.04.2023 US 202363498331 P; 01.04.2024 US 202418623291
(71) Applicant: Pacesetter, Inc., Sylmar, CA 91342 (US)
(72) Inventor: JIN, Maobing, Sylmar, CA 91342 (US)
(74) Representative: Barker Brettell Sweden AB

(57) **Abstract**

A healthcare system (100) comprises memory configured to store program instructions, a first device data translator (DDT) (214; 214a), a second DDT (214; 214b), and one or more processors. The first DDT (214; 214a) is configured to process data from a first type of medical device (124; 124a). The second DDT (214; 214b) is configured to process data from a second type of medical device (124; 124b) that is different from the first type of medical device (124; 124a). The one or more processors that, when executing the program instructions, are configured to receive patient data, the patient data comprising physiological data and at least one corresponding unique device identifier (ID) associated with a first medical device (124), wherein the first medical device (124) is configured to acquire the physiological data from a patient (108), determine whether the unique ID is associated with the first DDT (214; 214a) or the second DDT (214; 214b), and route patient data associated with the first medical device (124) to the first DDT (214; 214a) or the second DDT (214; 214b) that is associated with the unique ID.

## Description

### TECHNICAL FIELD

Embodiments of the present disclosure generally relate to methods, devices, and systems for receiving data from medical devices distributed globally, processing the data, and storing and/or providing the processed data to the end user.

### BACKGROUND

There are an increasing number and variety of patient devices in the world, producing huge amounts of raw device data that needs to be securely collected and processed without compromising the patient's privacy. For example, implantable devices that are implanted wholly or partially within a patient transmit data wirelessly to a device such as a phone or dedicated device, that then transmits the data over the internet to be collected and processed. Other devices, such as a Holter monitor, are not implanted but collect biological information from the patient that needs to be collected and processed.

Different devices acquire different types of data that require processing in different manners. Some devices become obsolete, new devices become available, devices are upgraded, and multiple versions of devices can be utilized that all transmit data that needs to be collected and processed. Many of these changes require significant changes on the processing end.

Further, multiple manufacturers or venders produce a variety of products that produce data that needs to be collected and processed. A single patient may have an implantable device, such as a defibrillator, of a first manufacturer, and a second device, such as a glucose monitor, of a second manufacturer. Currently the data is collected and processed by different vender systems that need to be maintained and upgraded separately, as well as programmed to interface with many different end user healthcare systems. These factors are some of the issues that compound the problem of collecting the data and processing it properly so that a report for a healthcare professional or clinician can be generated.

Therefore, the need exists for an integrated system to receive data collected by medical devices from multiple different types of devices and venders, process the data, and provide the appropriate information to the healthcare professional.

### SUMMARY

In accordance with embodiments herein, a healthcare system comprises memory configured to store program instructions, a first device data translator (DDT) and a second DDT, and one or more processors. The first DDT is configured to process data from a first type of medical device, and the second DDT is configured to process data from a second type of medical device that is different from the first type of medical device. The one or more processors that, when executing the program instructions, are configured to i) receive patient data, the patient data comprising physiological data and at least one corresponding unique device identifier (ID) associated with a first medical device, wherein the first medical device is configured to acquire the physiological data from a patient, ii) determine whether the unique ID is associated with the first DDT or the second DDT, and iii) route patient data associated with the first medical device to the first DDT or the second DDT that is associated with the unique ID.

Optionally, the unique ID includes at least one of i) a model number, ii) a serial number, iii) a unique key, iv) a second unique key, v) medical device type, or vi) type of medical data to be processed.

Optionally, the one or more processors are configured to compare the unique ID to a global device index to determine a medical device type or a type of medical data to be processed.

Optionally, the memory is configured to store a routing cache based on device model numbers or type of medical data to be processed, the routing cache configured to identify which of the first or second DDT processes the associated patient data, and wherein the one or more processors are configured to compare the unique ID to the routing cache.

Optionally, the one or more processors are configured to route the patient data to a queue configured to feed an associated one of the first DDT or the second DDT, wherein the queue is configured to temporarily store the patient data for the associated DDT.

Optionally, the one or more processors are further configured to route processed data from the first DDT to one of a report generator or a file generator, and route processed data from the second DDT to one of the report generator or the file generator.

Optionally, the one or more processors are further configured to route processed data from the first and second DDTs to a queue configured to feed an associated one of a report generator or a file generator, wherein the queue is configured to temporarily store the processed data for the associated one of the report generator or the file generator.

Optionally, the one or more processors are further configured to generate an electronic health record (EHR) based on processed data output by the first and second DDTs, and output the EHR.

Optionally, the system further comprises a third DDT configured to process data from a third type of medical device that is different from the first and second types of medical devices.

Optionally, the system further comprises a third DDT configured to process data from the first type of medical device.

Optionally, the system further comprises a transmitter, wherein the one or more processors are configured to generate a report or a file based on processed data output by at least one of the first and second DDTs, and the transmitter is configured to transmit the report or file to i) a predetermined electronic address associated a medical device that is associated with the processed data, ii) a location associated with a type of data in the report or file, or iii) a location associated with a type of medical device that is associated with the processed data.

Optionally, the system further comprises a second memory configured to store processed patient data and electronic health records, wherein the second memory is configured to be accessed by a clinician application.

In accordance with embodiments herein, a computer implemented method comprises utilizing one or more processors that, when executing specific program instructions, perform at least one of i) receiving patient data, the patient data comprising physiological data and at least one corresponding unique device identifier (ID) associated with a first medical device, wherein the first medical device is configured to acquire the physiological data from a patient, ii) determining whether the unique ID is associated with a first DDT or a second DDT, wherein the first DDT is configured to process data from a first type of medical device and the second DDT is configured to process data from a second type of medical device that is different from the first type of medical device, and iii) routing patient data associated with the first medical device to the first DDT or the second DDT that is associated with the unique ID.

Optionally, the routing further comprises routing the patient data to a queue configured to feed an associated one of the first DDT or the second DDT, wherein the queue is configured to temporarily store the patient data for the associated DDT.

Optionally, the routing further comprises routing the patient data to one of two queues configured to feed the first DDT, wherein each of the two queues are configured to temporarily store the patient data for the associated DDT.

Optionally, the method further comprises, responsive to receiving the patient data, outputting processed data with the first DDT and routing the processed data to a business service configured to output at least one of a file or a report.

Optionally, the method further comprises, responsive to receiving the patient data, outputting processed data with the first DDT and routing the processed data to a queue associated with one of a report generator or a file generator.

Optionally, the method further comprises, i) responsive to receiving the patient data, storing the patient data in a memory, ii) responsive to the DDTs outputting processed data, storing the processed data in a second memory, and iii) responsive to receiving a request for either the patient data or the processed data, transmitting the patient data or the processed data to a location remote from the one or more processors.

Optionally, the method further comprises adding a third DDT configured to receive the patient data, the patient data comprising the unique ID associated with the third DDT, wherein the third DDT is configured to process data that is different from the first and second types of medical devices.

Optionally, the method further comprises, a) responsive to the DDTs outputting processed data, storing the processed data in a memory, b) processing at least a portion of the processed data stored in the memory based on at least one of artificial intelligence, machine learning, and data analytics, and c) responsive to storing the further processed data in the memory: i) output, to a location remote from the one or more processors, recommendations for setting changes associated with one of the first or second medical devices, ii) output, to a location remote from the one or more processors, instructions to implement programmed settings associated with one of the first or second medical devices, iii) output, to a location remote from the one or more processors, instructions to automatically remotely program one of the first or second medical devices, and/or iv) output, to a location remote from the one or more processors, an alert.

### BRIEF DESCRIPTION OF THE DRAWINGS

Figure 1 illustrates a healthcare system that is deployed to multiple regions for the sake of patient data privacy requirements and adherence in accordance with embodiments herein.
Figure 2 illustrates a regional healthcare system that includes the data processing system that receives or collects data from multiple patient medical devices, and processes the data, in accordance with embodiments herein.
Figure 3 illustrates a diagram showing the data flow within the data processing system in accordance with embodiments herein.
Figure 4 illustrates the process flow of the data processing system for receiving patient data, routing the patient data, processing the patient data and outputting the results in accordance with embodiments herein.
Figure 5 illustrates an exemplary data processor server that is configured to generate reports and/or files from a particular type of medical device and/or a particular type of data in accordance with embodiments herein.
Figure 6 illustrates the process flow for receiving and processing a device data file from a medical device and outputting the results in accordance with embodiments herein.
Figure 7 illustrates a simplified block diagram of a system including an implantable medical device operated in accordance with embodiments herein.
Figure 8 shows a block diagram of the system associated with an implantable medical device in accordance with embodiments herein.
Figure 9 illustrates a healthcare system interfacing with a plurality of networked devices formed in accordance with embodiments herein.
Figure 10 illustrates a distributed healthcare system that can be included in one of the regional systems in accordance with embodiments herein.

### DETAILED DESCRIPTION

It will be readily understood that the components of the embodiments as generally described and illustrated in the Figures herein, may be arranged and designed in a wide variety of different configurations in addition to the described example embodiments. Thus, the following more detailed description of the example embodiments, as represented in the Figures, is not intended to limit the scope of the embodiments, as claimed, but is merely representative of example embodiments.

Reference throughout this specification to "one embodiment" or "an embodiment" (or the like) means that a particular feature, structure, or characteristic described in connection with the embodiment is included in at least one embodiment. Thus, appearances of the phrases "in one embodiment" or "in an embodiment" or the like in various places throughout this specification are not necessarily all referring to the same embodiment.

Furthermore, the described features, structures, or characteristics may be combined in any suitable manner in one or more embodiments. In the following description, numerous specific details are provided to give a thorough understanding of embodiments. One skilled in the relevant art will recognize, however, that the various embodiments can be practiced without one or more of the specific details, or with other methods, components, materials, etc. In other instances, well-known structures, materials, or operations are not shown or described in detail to avoid obfuscation. The following description is intended only by way of example, and simply illustrates certain example embodiments.

The methods and systems described herein may employ structures or aspects of various embodiments (e.g., systems and/or methods) discussed herein. In various embodiments, certain operations may be omitted or added, certain operations may be combined, certain operations may be performed simultaneously, certain operations may be performed concurrently, certain operations may be split into multiple operations, certain operations may be performed in a different order, or certain operations or series of operations may be re-performed in an iterative fashion. It should be noted that other methods and systems may be used in accordance with an embodiment herein. Further, wherein indicated, the methods and systems may be fully or partially implemented by one or more processors of one or more devices or systems. While the operations of some methods and systems may be described as performed by the processor(s) of one device, additionally, some or all of such operations may be performed by the processor(s) of another device described herein.

### Terms

The term "region" shall mean an area, defined by geography, politics, etc., such as a state, a country, an area, and/or a collection of countries. For example, the United States (US), the European Union (EU) (including at least some of the countries within the EU), and China can each be different regions. Optionally, other areas, such as areas outside the EU, may be included in the EU region, for example. Each region can be associated with a different set of patient data privacy requirements.

The term "virtual device engine" (VDE) as used herein refers to a simulation, or computer model stored within a cloud, network, or the like that can be utilized to represent a device, such as but not limited to, a medical device. The VDE can be utilized to replicate or simulate the operation of the device, including how an update, change, etc., to a programming session or set of instructions will occur at a device prior to the update, change, etc., being communicated to the device. In this manner the VDE can be utilized to modify the update, change, etc., to be communicated to the device based on operations associated with the VDE.

The term "programmer" as used herein refers to any and all electronic devices utilized by a clinician, technician, physician, caregiver, nurse, doctor, or the like. The electronic device can be a central processing unit (CPU), desktop computer, laptop computer, tablet, smartphone, smart watch, monitor console, etc. The electronic device includes one or more processors configured to follow instructions, and a transceiver configured to communicate over a network, in a cloud, wirelessly, over the air, through a wire, or the like. In one example, the clinician programing device communicates with a patient device and at least one system, e.g., healthcare system and data processing system.

The term "engine" as used herein refers to any processor, electronic device with a processor, server, or the like within a cloud, network, etc., configured to communicate with electronic devices via a network, mesh network, wirelessly, wired, or the like that can make determinations.

The term "patient data privacy (PDP) requirements" and "PDP" shall mean requirements regarding management and distribution of patient data, where such requirements are defined by a government, state, sovereign entity, agency, administrative body, and the like, The PDP requirements include a plurality of protected healthcare information (PHI) and/or a plurality of personal identification information (PII). The PHI and PII may be different from one region to the next. For example, the US has the Health Insurance Portability and Accountability Act (HIPAA) to protect sensitive patient health information from being disclosed without the patient's consent or knowledge. Within HIPAA, PHI and PII include a number of pieces of private and identifying information that may allow the person/patient to be identified and/or traced. Other countries may have similar privacy laws, such as the General Data Protection Regulation (GDPR) of the EU, the Personal Information Protection and Electronic Documents Act (PIPEDA) of Canada, and the like. The associated patient data cannot be accessed directly by other regions. It should be understood that more or fewer identifiers may be included in HIPAA as well as in the PDP requirements of other regions / countries.

The term "data processor", "data processors", "data processor server", "device data translators", "DDT", and "DDT server" shall mean a processor that processes and/or translates patient medical and/or physiological data into a format that can be further parsed to generate a file or report. Each DDT can be configured to process data from a particular medical device or physiological data of a particular type or types, and thus a healthcare system that processes data from medical devices may include many DDTs.

The terms "physiologic data", "physiological data" and "medical data" shall mean one or more of physiologic conditions or traits of the patient. For example, the physiologic data may represent cardiac activity signals (CA signals) sensed by electrodes positioned within or about the heart. The CA signals may also be sensed by electrodes provided on the housing of the IMD. As another example, the physiologic data may represent impedance signals, respiratory signals, heart sounds, cardiac activity, blood glucose data, pulmonary artery pressure, pulse oximetry, nerve activity (e.g., as measured within the spinal column or dorsal root), brainwave activity and the like. The physiologic data may represent pulse oximetry signals, cholesterol related information, blood sugar levels, and the like. Different types of physiological data can also be referred to as different types of data.

The term "device data" shall mean identification data that identifies a medical device. The device data can include the model number, serial number, an indication of the type of device, and/or an indication of the type of medical data collected and transmit, of which there can be multiple types.

The term "healthcare system" shall mean a system that includes a central service center and two or more regional systems, wherein each of the regional systems is associated with a region. In some cases, a region can have a distributed system such that there are two or more regional systems integrated together but physically separate from each other. The components and modules that make up the healthcare system and the regional systems can be distributed.

The terms "medical device", "patient device", "candidate device", "registered device", and/or more generally "device" shall mean any device, implantable (e.g., IMD) and non-implantable, that acquires and/or transmits medical information/data associated with a patient to a network. A non-implantable device can communicate with one or more implantable device and may also communicate with a network, such as the Merlin@Home^{™} device of Abbott Laboratories (headquartered in the Abbott Park Business Center in Lake Bluff, III.) or another device, such as a Holter monitor. In some cases, a patient can be associated with one or more implantable device and/or one or more non-implantable device.

The terms "unique device identifier" and "unique ID" shall mean one or more unique identifier associated with the medical device. The unique ID can be a device serial number, a device model number, a single code string, a birthdate of a patient, or any other indicator that is associated with the medical device. Each of the medical devices can be associated with one, two or more than two unique IDs.

The term "body generated analyte (BGA) test device" shall mean any and all equipment, devices, disposable products (implantable and/or non-implantable) utilized to collect and analyze a BGA. The BGA test device may implement one or more of the methods, devices and systems as described and referenced to in U.S. Patent Publication Number 2021-0020294 entitled "METHODS, DEVICES AND SYSTEMS FOR HOLISTIC INTEGRATED HEALTHCARE PATIENT MANAGEMENT" published January 21, 2021.

The term "IMD" shall mean an implantable medical device. Embodiments may be implemented in connection with one or more implantable medical devices (IMDs). Non-limiting examples of IMDs include one or more of neurostimulator devices, implantable leadless monitoring and/or therapy devices, and/or alternative implantable medical devices. For example, the IMD may represent a subcutaneous cardioverter defibrillator, cardiac monitoring device, pacemaker, cardioverter, cardiac rhythm management device, defibrillator, cardiac resynchronization device, neurostimulator, leadless monitoring device, leadless pacemaker, left atrial or pulmonary artery pressure sensor, other pressure sensor, blood glucose monitoring device, and the like. The IMD may measure electrical, mechanical, impedance, blood glucose, or pressure information. For example, the IMD may include one or more structural and/or functional aspects of the device(s) described in U.S. Patent Number 9,333,351, entitled "Neurostimulation Method And System To Treat Apnea" issued May 10, 2016 and U.S. Patent Number 9,044,610, entitled "System And Methods For Providing A Distributed Virtual Stimulation Cathode For Use With An Implantable Neurostimulation System" issued June 2, 2015, U.S. patent application 2023/0109023, entitled "System and Method for Intra-Body Communication of Sensed Physiologic Data", filed August 18, 2022. The IMD may monitor transthoracic impedance, such as implemented by the CorVue algorithm offered by St. Jude Medical. Additionally or alternatively, the IMD may include one or more structural and/or functional aspects of the device(s) described in U.S. Patent Number 9,216,285, entitled "Leadless Implantable Medical Device Having Removable And Fixed Components" issued December 22, 2015 and U.S. Patent Number 8,831,747, entitled "Leadless Neurostimulation Device And Method Including The Same" issued September 9, 2014. Additionally or alternatively, the IMD may include one or more structural and/or functional aspects of the device(s) described in U.S. Patent Number 8,391,980, entitled "Method And System For Identifying A Potential Lead Failure In An Implantable Medical Device" issued March 5, 2013 and U.S. Patent Number 9,232,485, entitled "System And Method For Selectively Communicating With An Implantable Medical Device" issued January 5, 2016. Additionally or alternatively, the IMD may be a subcutaneous IMD that includes one or more structural and/or functional aspects of the device(s) described in U.S. Patent Number 10,765,860, entitled "Subcutaneous Implantation Medical Device With Multiple Parasternal-Anterior Electrodes" issued September 8, 2020; U.S. Patent Number 10,722,704, entitled "Implantable Medical Systems And Methods Including Pulse Generators And Leads" issued July 28, 2020; U.S. Patent Number 11,045,643, entitled "Single Site Implantation Methods For Medical Devices Having Multiple Leads", issued June 29, 2021, U.S. published application US20210330239A1, entitled "Method and system for adaptive-sensing of electrical cardiac signals" filed March 4, 2021. Further, one or more combinations of IMDs may be utilized from the above incorporated patents and applications in accordance with embodiments herein. Embodiments may be implemented in connection with one or more subcutaneous implantable medical devices (S-IMDs). For example, the S-IMD may include one or more structural and/or functional aspects of the device(s) described in U.S. Patent Number 10,722,704, entitled "IMPLANTABLE MEDICAL SYSTEMS AND METHODS INCLUDING PULSE GENERATORS AND LEADS", issued July 28, 2020; U.S. Patent Number 10,765,860, entitled "SUBCUTANEOUS IMPLANTATION MEDICAL DEVICE WITH MULTIPLE PARASTERNAL-ANTERIOR ELECTRODES", issued September 8, 2020. In another example, the IMD 12 may be an ICM that includes one or more structural and/or functional aspects of the device(s) described in U.S. Patent 9,949,660, filed March 29, 2016, entitled, "Method And System To Discriminate Rhythm Patterns In Cardiac Activity". The IMD may represent a passive device that utilizes an external power source, an entirely mechanical plan will device, and/or an active device that includes an internal power source. The IMD may deliver some type of therapy/treatment, provide mechanical circulatory support, and/or merely monitor one or more physiologic characteristics of interest (e.g., PAP, CA signals, impedance, heart sounds).

The terms "IMD data", "patient device data", and "patient data" shall refer to any and all types of information and signals conveyed from an implantable, semi-implantable or external medical device to a local or remote external device. Nonlimiting examples of IMD data include cardiac activity signals (e.g., intracardiac electrogram or IEGM signals), impedance signals (e.g., cardiac, pulmonary or transthoracic impedances), accelerometer signatures (e.g., activity signals, posture/orientation signals, heart sounds), pulmonary arterial pressure signals, MCS rpm levels, MCS flow rates, device alerts and the like.

The terms "MP" and "clinician" shall mean medical personnel, nonlimiting examples of which include doctors, nurses, hospital or clinical staff, pharmacist, physical therapist, and any other person trained or licensed to provide medical assistance to a patient.

The terms "transmit", "transmitting", "obtain" and "obtaining", as used in connection with data, signals, information and the like, shall mean communications between one device and at least one of another device or system, and include at least one of i) accessing memory of an external device or remote server where the data, signals, information, etc., are stored, ii) receiving the data, signals, information, etc., over a wireless communications link between the IMD and/or medical device and a local external device, and/or iii) receiving the data, signals, information, etc., ata remote server over a network connection. The obtaining operation, when from the perspective of an IMD, may include sensing new signals in real time, and/or accessing memory to read stored data, signals, information, etc., from memory within the IMD. The transmitting or obtaining operation, when from the perspective of a local external device, includes receiving the data, signals, information, etc., at a transceiver of the local external device where the data, signals, information, etc., are transmitted from an IMD and/or a remote server. The transmitting or obtaining operation may be from the perspective of a remote server, such as when receiving the data, signals, information, etc., at a network interface from a local external device and/or directly from an IMD. The remote server may also obtain the data, signals, information, etc., from local memory and/or from other memory, such as within a cloud storage environment and/or from the memory of a workstation or clinician external programmer.

The terms "communication", "communications", "query", "application programming interface (API) call", "API call response", and "request" shall be interchangeable and shall mean that uni-directional and/or bi-directional communication is occurring between modules, regional systems, the central service center, medical devices, etc.

The terms "clinician application" and "patient application" shall mean interfaces provided on a plurality of electronic devices that are configured to receive patient data, medical device data, and/or requests that are entered by the patient / clinician. The electronic devices may include, but are not limited to, smart phones, desktop or laptop computers, tablet devices, smart TVs, fixed cameras, smart watch, wearable heart rate monitor, portable or handheld cameras, recording devices, personal digital assistant (PDA) devices, and the like. The electronic device may include a graphical user interface, through which the patient or another user enters the patient and/or device data. For example, a user may use a keyboard, touch screen, camera, microphone, and/or mouse to enter patient data.

The terms "processor," "a processor", "one or more processors" and "the processor" shall mean one or more processors. The one or more processors may be implemented by one, or by a combination of more than one implantable medical device, a wearable device, a local device, a remote device, a server computing device, a network of server computing devices and the like. The one or more processors may be implemented at a common location or at distributed locations. The one or more processors may implement the various operations described herein in a serial or parallel manner, in a shared-resource configuration and the like.

In accordance with embodiments herein, the methods, devices, and systems may be implemented in connection with methods, devices, and systems described in U.S. patent application number 2023/0054317, filed on June 07, 2022, titled "System and Method for Implantable Medical Device Remote Programming", U.S. patent application number 2023/0414951, filed on June 19, 2023, titled "System and Method for Implantable Medical Device Remote Programming", and U.S. patent application number 2024/0013895, filed on May 1, 2023, titled "Global Indexing System for Maintaining Patient Data Privacy Requirements for Medical Devices and Associated Patients".

### System Overview

In accordance with new and unique aspects herein, systems and methods are described that receive patient data from medical devices located globally and route the data to a data processing server that is referred to herein as a device data translator (DDT) or DDT server. The data is routed to a particular DDT based on the type of medical device and/or the type of data collected by the medical device. For example, each time data is received from a defibrillator, the data is routed to a DDT that processes the defibrillator data, and when data is received from a pulmonary arterial sensor, the data is routed to a different DDT that processes the data collected by the pulmonary arterial sensor. Report(s) and/or file(s) can be generated to send the required/requested information to the healthcare professional, a remote care application, a patient application, a storage accessible by external parties, a storage accessible by the healthcare system, and the like. Therefore, the processed information can be pushed out to a client and/or patient, and/or made available within a database.

The systems and methods described herein concern novel uses of, and improvements to, the technology or technical field of the receipt, routing, processing and storage of medical device data and protected patient data related to medical devices while adhering to patient data privacy (PDP) requirements that restrict access to a patient's data. Some patients travel around the world and the embodiments described herein discuss how the data can be acquired and processed while maintaining PDP requirements.

Systems and methods herein provide the improvements and practical application to provide regional systems with the ability to receive data from multiple medical devices, from multiple venders, and process the data efficiently. In contrast, conventional systems cannot process data from multiple venders, resulting in increased complexity and confusion for the practitioner and the patient. A further improvement is realized as the systems can be maintained efficiently by processing different types of data / data from different types of devices with different DDTs. Processing capacity can easily be increased or decreased depending on demand, new processing capability can be added without disrupting current processing of other device data, and efficiencies are realized as a healthcare professional receives information for multiple devices and/or multiple patients through a single system.

Accordingly, a technical advantage of the systems and methods described herein is the ability to receive data globally, route the data to an identified processing server for processing, and provide the results of the processed data to the healthcare professional. A further technical advantage is the ability to deliver a particular treatment for medical conditions experienced by patients. For example, the patient's own data can be processed and/or compared / combined with similar data, such as in a clinical study and/or by using Al, machine learning, etc., to provide improved settings, parameters, and/or recommendations to improve patient health, address symptoms and conditions being experienced by the patient, educate the patient, encourage positive behavior modification, and the like. This improves the technology or technical fields of patient monitoring and the medical treatment of patients by addressing patient conditions and symptoms, and by transforming a patient's medical state from an abnormal or undesirable state to a normal or desirable state.

### Globally Distributed Patient Device Collection and Processing System

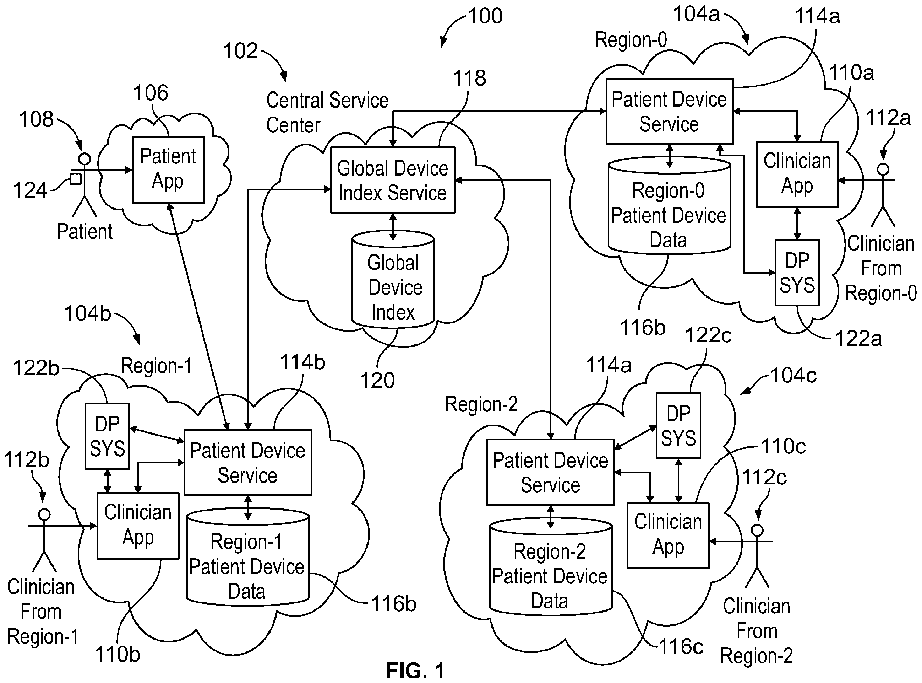

Figure 1 illustrates a healthcare system 100 that processes patient data and is deployed to multiple regions for the sake of patient data privacy (PDP) requirements and adherence in accordance with embodiments herein. The healthcare system 100 provides the ability to receive and process data efficiently within a patient's assigned region. In some embodiments, the patient data can be received though the cloud associated with a different region, and the system 100 determines the proper routing for the patient data. The data can be of different types and can be received from different types of medical devices that can be provided by different manufacturers or vendors.

The healthcare system 100 includes a central service center 102 and a plurality of regional systems 104. The central service center 102 includes one or more processors, one or more storage devices (e.g., memory, etc.), and modem(s)/transmitter(s)/transceiver(s) to electronically communicate with two or more regional systems 104. Regional system 0 104a may be, for example, located in a first region such as China. Regional system 1 104b can be located in a second region, such as the European Union (EU), while regional system 2 104c can be located in a third region, such as the United States (US). In some embodiments, there can be two regional systems 104, while in other embodiments there can be more than three regional systems 104.

The central service center 102 includes a global device index service 118 (e.g., engine) that can communicate with, and receive and process queries from, the regional systems 104. The global device index service 118 uses the one or more processors to build and access a global device index 120 that provides a centralized index for the healthcare system 100, facilitating the addition of newly entered devices, the modification of existing device records, and the movement of protected patient data and device data from one region to another. The global device index 120 includes at least one of i) one or more unique device identifiers (ID) associated with each of the medical devices (e.g., model number, serial number, device type), ii) the region where the medical device is registered, if applicable, iii) the status of the medical device, iv) when and/or who last updated the medical device record, and v) when and/or who created the medical device record. In some embodiments, the one or more unique ID may be associated with a medical device that includes a plurality of devices, such as an IMD that has two or more separate stimulation and/or monitoring elements. The central service center 102 does not store patient data associated with a medical device (e.g., PHI and/or PII), and can answer queries such as, but not limited to, i) is the medical device data valid, ii) is the medical device registered, iii) is the medical device active, and iv) in which region is the medical device being used. The global device index can be stored as a single database or distributed among different files and/or memory locations while still being integrated together and functioning as a single database and/or index.

Each of the regional systems 104 can include one or more processors and modem(s)/transmitter(s)/transceiver(s) to electronically communicate with a plurality of patient applications 106, clinician applications 110 and the central service center 102. The plurality of clinician applications 110 are associated with one regional system 104 and accept input and requests from clinicians 112 located within the same region. For example, the clinician applications 110a accept inputs only from the clinicians 112a located in and/or assigned to the regional system 104a to protect PDP requirements. There can be many clinician applications 110 within the same region, located at doctor offices, hospitals, medical facilities, loaded on a clinician's device (e.g., phone, computer, etc.), and the like.

A patient 108 can have at least one medical device 124 that acquires physiological data and/or other medical-based data associated with the patient 108. The medical device 124 can be implanted within the patient 108, semi-implanted (e.g., at least portions attached to, in communication with, and/or extending into the skin of the patient 108), or external with respect to the skin of the patient 108. The medical device 124 can communicate, in one direction or bi-directionally, via the patient application 106 or any device and/or transmitter/transceiver discussed further herein, with a data processing system 122 within the regional system 104 that the patient 108 / medical device 124 is assigned to. In some cases, the medical device 124 can communicate directly with the regional system 104. Although not individually shown, it should be understood that there may be many thousands of patients 108 in and/or associated with each regional system 104, each of the patients 108 having at least one medical device 124.

The regional systems 104 further each include a patient device service 114 using the one or more processors to build and/or access a regional patient device database 116 that stores one or more unique ID (e.g., one or more key) that is associated with a particular medical device 124 (e.g., IMD, non-implantable medical device, etc.). The one or more unique ID can be model and serial number, a single code string assigned to a medical device, more than two unique IDs, a model number and a birthdate, the type of data received from the medical device 124, and the like. The medical devices 124 can be associated with different venders, and thus the healthcare system 100 is advantageously not limited to medical devices 124 associated with a single vender and is not limited to any particular type of patient care (e.g., cardiac, pulmonary, monitoring elements within blood and/or tissues) or limited to any particular type of data (e.g., EGM, EKG, heart sounds, blood pressure, blood glucose level). The regional patient device database 116 can also store patient specific information that is protected, such as PHI and PII.

Each of the regional systems 104 includes one or more storage device (e.g., memory, etc.) for storing the regional patient device database 116, which can be stored as a single database or distributed among different files and/or memory locations while still being integrated together and functioning as a single database and/or index. Each of the regional systems 104 has access only to the medical device information assigned to that particular region, and cannot access a different region's patient device database 116 to review or exchange data, and the like.

The global device index service 118 provides global application programming interface (API) support for queries from and to the regional systems 104. For example, when data is received from a patient application 106 or a clinician application 110, the patient device service 114 may query the global device index 120 to ensure that the device data is assigned to that particular regional system 104. If the medical device 124 cannot be identified or is assigned to a different regional system 104, the data will not be processed and stored in the regional system 104. In some cases, an error message will be generated and/or the data can be transmitted to the central service center 102 for proper routing. In some embodiments, the patient application 106 may transmit data through the cloud to the central service center 102 for routing to the assigned regional system 104.

If the medical device 124 is assigned to the regional system 104, the data is sent to the data processing system 122. The data processing system 122 identifies the type of data and/or the device type to determine where to send the data for processing, as discussed further herein. The raw data sent by the medical device, as well as processed data, reports, files, etc., generated by the data processing system 122 are stored in one or more memories and/or databases (not indicated). The data processing system 122 can send some or all of the processed data, reports, files, and/or notifications related to the data to the clinician application 110, the patient application 106, or another storage location such as an FTP server. Additionally or alternatively, the clinician 112 and/or clinician application 110 can access and/or request the information stored in the one or more database, such as by sending an API.

In some embodiments, the patient application 106 and/or medical device 124 can be configured to communicate with the central service center 102. The global device index service 118 or other routing system can determine which region the medical device 124 is assigned to, and direct the patient application 106 and/or medical device 124 to communicate with the appropriate region. Optionally and/or alternatively, the central service center 102 can convey the patient data to the appropriate regional system 104, which may be an advantage as the patient 108 may be traveling outside of their home region when their data is uploaded. In some embodiments, the global device index service 118 can also determine the appropriate routing needed within the regional system 104, such as to transmit the data to a desired data processor, or type of data processor or DDT, as discussed further below.

Although the elements within the central service center 102 and the regional systems 104 are depicted as separate, it should be understood that functionality may be interrelated. For example, some aspects of the patient device service 114 can be incorporated within the data processing system 122. Further, elements within the healthcare system 100 can be distributed.

### Globally Distributed Patient Device Data Collection and Processing System

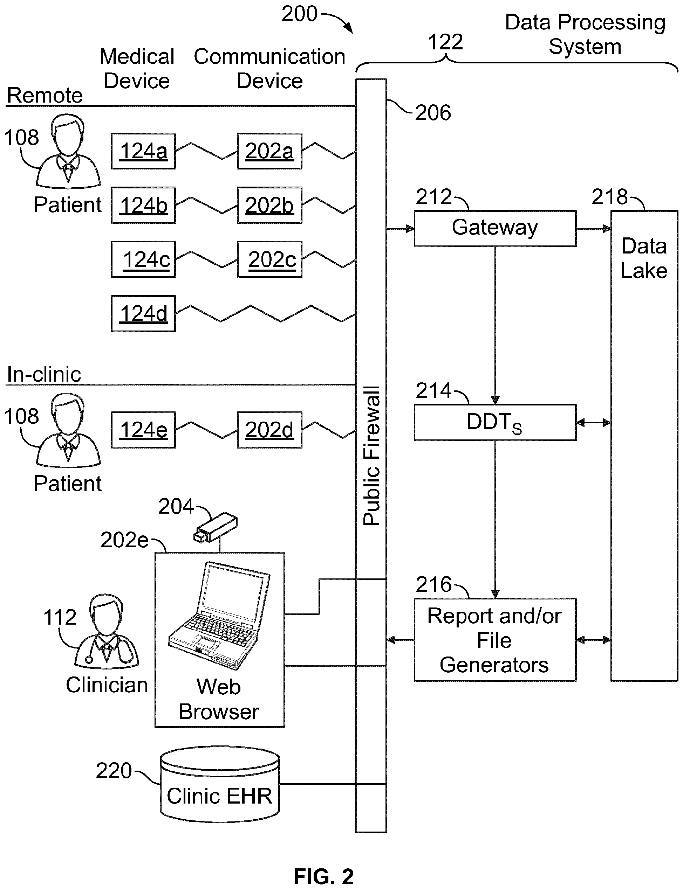

Figure 2 illustrates a regional healthcare system 200 that includes the data processing system 122 that receives or collects data from multiple patient medical devices 124, and processes the data, in accordance with embodiments herein. The data processing system 122 receives device identification data, identifies the device, type of device, and/or type of data collected, and creates the appropriate routing and workflow for processing the received medical data (e.g., physiological data, data collected from the patient 108). Although not explicitly shown, it should be understood that each component of the system 200 can include at least one memory storing program instructions and one or more processors configured to execute the program instructions.

Patients 108, and thus patient medical devices 124, are located remote from the data processing system 122. A plurality of medical devices 124, that are assigned to the same region are shown on Figure 2. The medical devices 124 can be implanted, such as pulmonary arterial (PA) pressure sensor, implantable cardiac device (ICD), pacemaker, implantable medical device (IMD), implantable cardiac monitor (ICM), etc., partially implanted, such as a continuous glucose monitor, and/or acquire data from the patient externally, such as a Holter monitor, EKG machine, glucose monitor and/or blood pressure monitor with network capability, etc. The patient medical devices 124 can be associated with different venders or manufacturers, and thus the data processing system 122 is advantageously not limited to medical devices 124 associated with a single vender and is not limited to any particular type of patient care (e.g., cardiac, pulmonary, monitoring elements within blood and/or tissues, etc.).

The medical devices 124 can be configured to directly transmit the data wirelessly over the internet and/or to the cloud, over any known network, cellular, POTS, satellite, and may be part of the Internet of Things. In other cases, the medical devices 124 transmit, via Bluetooth, GSM, and/or other transmission protocols or cables, the medical data and device data to a communication device 202 such as a phone, tablet, programmer, computer, transmitter, transceiver, dedicated console, laptop, desktop computer, cpu, smartphone, smart device, etc., which conveys the data to the internet. In some embodiments, the communication device 202, and/or the medical device 124 if external to the patient 108, can be hardwired to a network configured to communicate to the data processing system 122. The communication device 202 can function as a transceiver, and thus can communicate bi-directionally, sending data to and receiving data from the data processing system 122. The communication device 202 can be or can include the patient application 106. Data transmitted can include, but is not limited to, device identification information such as one or more unique ID (e.g., one or more key) that is associated with a particular medical device 124 (e.g., IMD, non-implantable medical device, etc.). The one or more unique ID can be model and/or serial number, a single code string assigned to a medical device 124, more than two unique IDs, a model number and a birthdate, and the like. The device data can also include indication of the one or more type of data transmitted by the medical device 124. The transmitted data also includes physiological data collected from the patient 108 (EEG, EKG, heart rate, blood pressure, blood sugar levels, etc.). If the medical device 124 is external to the patient 108, the communication device 202 and the medical device 124 may be integrated together, such as a Holter monitor that has integrated ability to transmit data over the internet.

The communication device 202 can passively receive data from the medical device 124, interrogate the medical device 124 on demand or on a schedule, etc. By way of example only, medical device 124a can be a sensor, such as a PA pressure sensor, that is interrogated by an external interrogator (e.g., communication device 202a) that then transmits information over the internet to the regional data processing system 122. The medical device 124b may be an implantable device (e.g., ICD, IMD, ICM) that conveys information to a home patient monitoring system (e.g., communication device 202b), which then transmits the information over the internet, POTS, or other network to the regional data processing system 122. The medical device 124c can be another implantable, partially implantable, and/or external device that communicates with a patient application 106 on a phone, tablet, and the like (e.g., communication device 202c). The medical device 124d can be a medical device that transmits directly to the internet. In an in-clinic setting, the medical device 124e can communicate with a communication device 202d such as programmer, that may be operated by a clinician 112. In some cases, the clinician 112 can input data into a communication device 202e, and also can manually move patient physiological and other data from one communication device 202 or other device that has collected medical data, over a network or a portable drive 204 (e.g., sneakernet), and send the data to the data processing system 122.

Transmissions from the medical device 124 and/or communication device 202 can be expected randomly or on a daily, weekly, bi-weekly, or other upload schedule. In some embodiments, the physiological data is collected from the patient 108 once a day via an at-home unit, patient device application, etc., and packaged into a file format that contains a unique device data file for each data collection.

For example, if the medical device 124 is an IMD that concerns cardiac monitoring, pacing, defibrillation and the like, the following data may be collected daily: i) daily percentage pacing trend, ii) daily activity trend, iii) daily atrial tachycardia/atrial fibrillation (AT/AF) frequency trend, iv) duration of AT/AF episodes trend, v) ventricular rate during atrial arrhythmia trend, and vi) percent pacing alerts such as percentage of ventricular pacing. It should be understood that many other types of data and alerts may be collected, based on the type of device, type of data collected, patient requirements, etc.

In some embodiments, the patient application 106 has a transmission frequency that is a scheduled weekly maintenance upload to the system 122. Each daily data collection from the medical device 124 can be small, approximately 11K bytes. The collection interval from the medical device 124 can be once daily which results in a maximum of 7 unique data collection device data files sent to the system 122 every week. The patient application 106 can package the data collections for the week into a zip file prior to transmission. In some cases, the system 122 processes all of the received transmissions at an equal priority level. Once received, the system 122 will process each daily data collection separately. In some cases, the data is used to trend each collection as a data point for visual-based display, included in reports, etc. The system 122 is able to process transmissions that include at least one device data file, as it is possible that a patient 108 may miss a collection interval due to being out of RF range of the patient application 106.

It should be understood that there can be many medical devices 124 for every data processing system 122. Also, to facilitate processing the large volume of data, providing back-up processing capability, and/or system redundancy, etc., a regional system 104, such as the regional system 104c that services patients located in the United States, can have multiple data processing systems 122 that can be physically and/or logically distributed. Also, components within the data processing systems 122 can be physically and/or logically distributed, replicated as needed for scale, removed for obsolescence, and the like.

The communication devices 202 and/or medical devices 124 (if capable of direct communication with the data processing system 122), transmit patient data to a predetermined electronic address or location on the internet. The data can enter through one or more firewall 206 and be directed to a patient device gateway 212. The gateway 212 comprises hardware, software (e.g., middleware), and/or firmware.

The gateway 212 identifies one or more of the medical device 124, the type of medical device 124, device model number, device serial number, manufacturer, version of device, version of device software, location of medical device 124 (e.g., assigned region), a first or second unique key, and/or the type of medical data transmitted by the medical device 124 and determines where the medical patient data should be routed to be processed. In some cases, the identifier(s) of the medical device 124, e.g., unique ID(s), are compared to the global device index 120 to determine a medical device type or a type of medical data to be processed.

The gateway 212 routes the data to a queue manager, discussed further herein, and the appropriate DDT 214. The raw data can be stored in one or more database, catalog, file structure, file system, memory, etc., in data lake 218.

In some embodiments, a first DDT 214 can process files received from patient applications 106, clinician applications 110, programmers and other communication devices 202, etc., such as via internet data transmissions. These may include manual sneakernet uploads via programmers or other interfaces or communication devices 202. Some transmissions can include one or more of stored EGM data, freeze capture data, trended data, impedance monitoring information, etc. Processing by the DDT 214 can include, but is not limited to, generating i) graphical user interface web application data files (e.g., GDF) that can be used by clinician applications 110 and patient applications 106, PDF reports for applications to display to users, IEEE data files for public health data sharing, PACEART data files for public health data sharing, and/or master data file (e.g., MDF) for clinical studies.

Once the data is processed by the DDT 214, the data can be routed to another queue manager and one or more report and/or file generator 216, and the processed data can be stored in the data lake 218. For example, processed data can include an Electronic Health Record (EHR) report based on processed data output by the DDT(s). The EHR report and other processed data and files can be stored in a database (e.g., memory) that is accessible by approved clinicians 112, pushed (e.g., output) to the clinician application 110, and/or securely pushed to a database of a clinic EHR 220.

The data lake 218 or other memories, memory systems or servers can store raw medical data, processed data, reports, historical data, user preferences, and the like. In some embodiments, one or more database can be used for handling and storing long strings of data, while another one or more database can be used to store data associated with one or a limited number of types of devices 124.

The DDT 214 can include or communicate with working and archive directories stored in the data lake 218, a local memory, or other memory, that stores all significant files used and generated during processing. Although other directories may be used, the working directories can include: i) configuration directory to store all server configuration data files, ii) incoming directory to provide a landing directory for incoming files, iii) in-process directory to hold incoming tarballs being processed, and iv) outgoing directory to hold processed files. Archive directories can also be included, such as: i) incoming directory to save all incoming transmission files, ii) intermediate directory to save all report generator and file generator 216 generated files, iii) failed directory to save all incoming tarballs or other structures which failed processing; and iv) outgoing directory to save all processed files.

Referring to the DDTs 214, in some embodiments, software referred to as a process manager can be a generic component installed on all the DDTs 214 within the data processing system 122, adding to the flexibility of the system 122. For example, at a high level the architecture of each of the DDTs 214 can be the same or similar, and different software, firmware, and/or hardware plug-ins can be incorporated for different outputs. The process manager is configurable at initialization to meet the unique requirements of the DDT 214 in which it executes. The process manager launches and manages processes to translate raw device data into meaningful data (e.g., in XML and JSON format) although other formats can be used based on the data, end user, desired reports, etc. The process manager generates the follow-up PDF report files (other formats can be used) from which the final reports provided to the clinician 112 will be generated. The process manager also ensures that the processed file is created for routing to the report and/or file generator 216. For example, in some embodiments the process manager configuration can be an XML based file which is simple to parse in Java as opposed to a variable length string-based configuration file.

As discussed herein, a plurality of DDTs 214 are provided, wherein at least one DDT 214 is configured to process data from each of the multiple different types of medical devices 124. In some embodiments, if a large amount of data is expected (e.g., many of the same type of medical device 124 is implanted within many patients 108), more than one DDT 214 is provided to process the data from a single type of medical device 124. Each of the DDTs 214 can be a server that is configured as either a dedicated physical host or a virtual host. The specificity of the processing capabilities of each of the DDTs 214 improves the flexibility of the data processing system 122, allowing easier additions and deletions based on the type of medical device 124, type of medical data, and volume of each, as well as upgrades and repairs of software, firmware, and/or hardware.

In contrast with previous systems, patient data from multiple different types of medical devices 124 was sent to a single processor for processing. Therefore, if the software for any of the devices 124 was upgraded, the processing ability may be limited. Also, upgrading the processor to add processing capability to process new types of devices and to remove the processing capability for obsolete devices is more cumbersome and disruptive than adding or removing a DDT 214 dedicated to a particular type of medical device 124 or type of data.

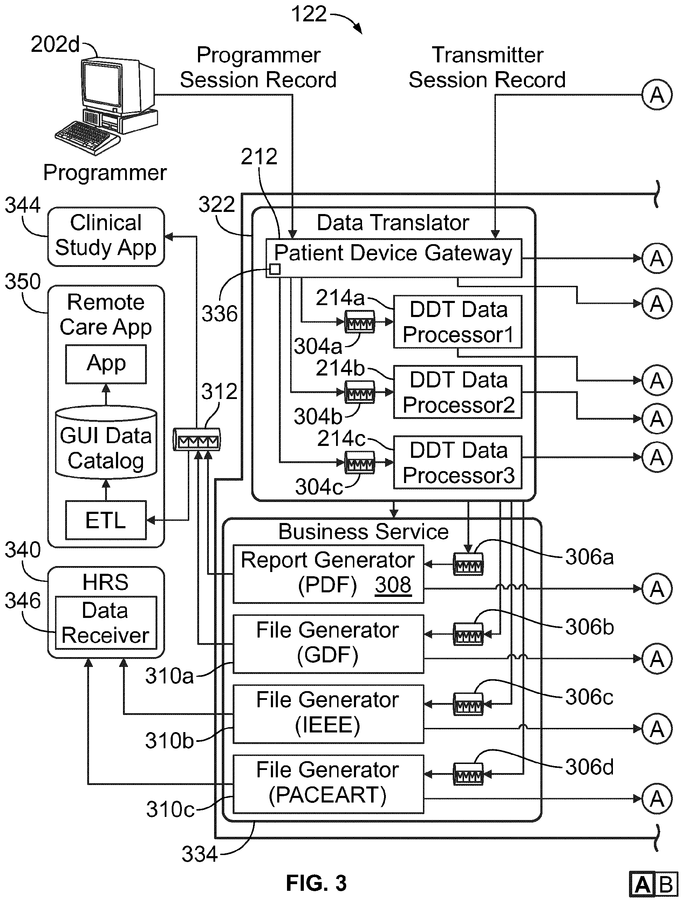

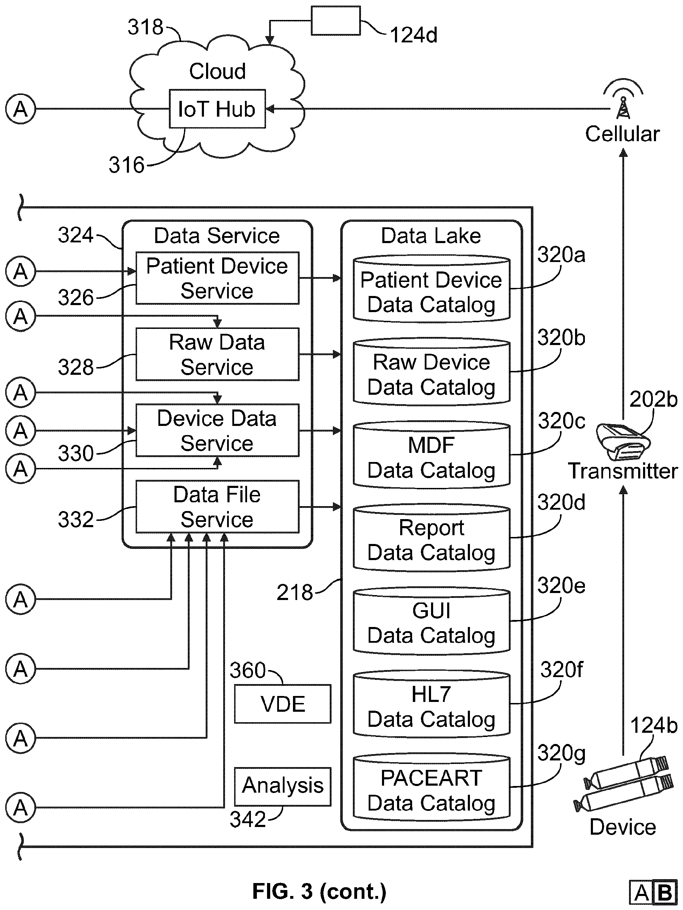

Figure 3 illustrates a diagram showing the data flow within the data processing system 122 in accordance with embodiments herein. It should be understood that the elements, modules, instances, etc., of the data processing system 122 can be distributed. There is no requirement to physically or logically locate elements in particular geographic locations. Additionally, a single region may utilize multiple gateways 212 to facilitate the data collection and processing from many medical devices 124. Although not explicitly shown, it should be understood that each component of the processing system 122 can include at least one memory storing program instructions and one or more processors configured to execute the program instructions.

The gateway 212 is a component that receives raw device data from medical devices 124 globally. For example, a distributed Internet of Things (IoT) hub 316 can receive the data. The loT hub 316 can be within the cloud 318 associated with the particular region. In some embodiments, patient data may be received by an loT hub 316 associated with another region and forwarded to the assigned region or loT hub 316 of the assigned region. The gateway 212 routes raw device data to one of the DDTs 214 based on the medical device's model number and/or other information discussed further herein. The gateway 212 supports pairing of each of the medical devices 124 with one or more DDT 214 that is configured to process that type of data. The processing can be referred to as asynchronous, wherein the data comes into one service (e.g., gateway 212) and is then routed to many services (e.g., DDTs 214a, 214b, 214c). As the gateway 212 is primarily identifying and routing the data, the gateway 212 processes each device data file received faster than the data is processed by the DDTs 214. For simplicity, only two medical devices 124b, 124d and two communication devices 202b, 202d are shown in Figure 3.

The DDTs 214 process the raw device data, for example, based on data type. The DDTs 214 can parse the raw device data, extract the patient device data, and in some embodiments, format the patient device data into a master data format (MDF). Although three DDTs 214 are shown, there may be less than three DDTs 214 or more than three DDTs 214. In general, the gateway 212, DDTs 214, and associated queues 304 can be referred to generally as data translator(s) 322.

In some embodiments, first and second DDTs 214 are configured to process data from first and second types of medical devices 124, wherein the second type of medical device 124 is different from the first type of medical device 124. One or more processors of the data processing system 122 are configured to receive patient data, the patient data comprising physiological data and at least one corresponding unique device identifier (ID) associated with a first medical device 124, wherein the first medical device 124 is configured to acquire the physiological data from a patient. The one or more processors are configured to determine whether the unique ID is associated with the first DDT 214 or the second DDT 214, and route patient data associated with the first medical device 124 to the first DDT 214 or the second DDT 214 that is associated with the unique ID. In other embodiments, a third DDT 214 is configured to process data from a third type of medical device 124 that is different from the first and second types of medical devices 124. In some cases, a first and third DDT 214 can be configured to process data from a first type of medical device 124.

Report generators 308 and file generators 310 can be referred to generally as business services 334. Each business service 334 can have one or more particular type of output that is based on the needs or requirements of the client or other external body that will receive and/or request the output data. In some embodiments, each business service 334 transforms the received data into another format. In some embodiments, the system 122 can include a transmitter configured to transmit a report or file to a predetermined electronic address associated a medical device 124, wherein the report or file are based on processed data output by one or more of the DDTs 214. In other embodiments, the report or file, processed data, etc., can be transmit to a location associated with the type of data in the report or file (e.g., data associated with a particular type of device, data associated with a particular type of signal), or to a location associated with the type of medical device that provided the original raw data. For example, in some cases, it may be advantageous to collect the data from a certain type of device to verify operation, quality, performance, etc., such as to confirm certain operations before and/or after a software modification.

Report generator 308 can create PDF report files and related information based on the MDF file of a patient device transmission record generated by the DDTs 214. One report generator 308 is shown; however, more than one report generator 308 can be included in the system 122. In some embodiments, the report generators 308 translate device data into other formats (e.g., XML and JSON format) and generate follow-up PDF reports similar to those output by the programmers used by the clinicians 112. In otherwords, the follow-up PDF reports can provide substantially similar data, and may be formatted in a substantially similar way, such as the reports clinicians 112 and other health care professionals are currently familiar with.

The file generator 310 analyzes the MDF file generated by the DDT 214 and creates a specific file for one supported type, such as GDF, IEEE/HL7, PACEART, CA, PDF, etc. In general, the file generators 310 convert intermediate device data from one format to another for one or more specific purpose. In some embodiments, the file generator 310 converts intermediate device data in XML format into a format which is readily used for other specific purposes such as generation of the IEEE file for export to the EHR system(s), such as the clinic EHR 220 of Figure 2 or a server/interface accessible to multiple venders and/or clinicians 112 such as but not limited to Heart Rhythm Society (HRS) 340. Although only HRS 340 is shown, it should be understood that there can be a plurality of different servers/interfaces associated with different groups and/or individuals, including but not limited to regulatory agencies, hospitals, universities, etc., receiving data from the system 122, and that at least some of the different servers/interfaces can be accessed by multiple venders, groups, individuals, and/or clinicians 112. In some embodiments, the file generator 310 may be configured to output two different types in support of a particular business need. Three file generators 310 are shown, but more or less than three file generators 310 can be included in the system 122.

One or more of the report generators 308 and file generators 310 are configured to transmit data to one or more remote care applications 350. The remote care application 350 can include but are not limited to the patient application 106 and the clinician application 110. For example, each of the medical devices 124 and/or communication devices 202 can have an associated predetermined electronic address that identifies where processed data, files, and/or reports should be transmitted. Other destinations can include servers and/or file storage locations that compile data for research, analysis, troubleshooting, quality control, etc.

Data services 324 includes a plurality of different services configured to store data in data lake 218. Different services can be provided for different functions, and more or less services than illustrated can be provided within the system 122. By way of example only, a patient device service 326 and raw data service 328 can receive data from the gateway 212. In some embodiments, the raw data can include all information from the medical device 124. Device data service 330 receives data from the DDTs 214, while data file service 332 receives data from the report generators 308 and file generators 310.

The data lake 218 stores raw and processed data received by and generated by the system 122, as well as reports and files generated by the system 122. The data lake 218 includes a plurality of databases 320 (indicated as databases 320a-320g) or other data stores, catalogs, or memories. In some embodiments, each of the databases 320 or catalogs can be associated with a particular medical device type and include all of the data associated with that device type. In some embodiments, the data stored in the data lake 218 can be used for business services 334 such as research, clinical trials, clinical study application 344, analysis module 342, for comparison with patient's outcome data, for determining clinical efficacy of a given feature, determination for proposed and/or automatically adjusted settings and/or parameters, patient remote care, clinical study in general, and the like.

The analysis module 342 can be processor(s) and/or server(s) implementing computer-implemented method(s) that use one or a combination of different models, techniques, statistical tools, Al algorithms, data analytics, and/or machine learning. For example, at least one of the following can be used: artificial neural networks, Monte Carlo analysis techniques, a Bayesian network, a statistical-based anomaly detection technique, one or more Markov models, knowledge-based techniques, neural networks, clustering and outlier detection, demographic analysis, genetic algorithms, and/or fuzzy logic techniques. In yet another example, an artificial intelligence algorithm is utilized that includes numerous variables and weights that are continuously adjusted to determine the sensor parameter.

The remote care application 350 and/or a remote web application are located remote from the data processing system 122 and can utilize the data stored in the databases 320 to provide patient education, behavior modification, suggest and/or directly implement treatment by changing programming settings in the medical device 124, support clinicians 112 and patients 108 with a variety of monitoring and treatment protocols, allow browsing by clinicians 112 of recent transmissions, support remote programming, support review of diagnostics, etc. For example, trended data, histogram data, ventricular pacing alerts, alerts associated with exceeding boundaries, etc., can be shown on the patient application 106 and/or clinician application 110. The data stored in the databases 320 can be used to support remote care, clinical study application 344, regulatory approval processes, etc. In some embodiments, the analysis module 342 can process at least a portion of the processed data stored in the memory based on at least one of artificial intelligence, machine learning, and data analytics, and responsive to storing the further processed data in the memory: i) output, to a location remote from the one or more processors, recommendations for setting changes associated with one of the first or second medical devices 124, ii) output, to a location remote from the one or more processors, instructions to implement programmed settings associated with one of the first or second medical devices 124, or iii) output, to a location remote from the one or more processors, instructions to automatically remotely program one of the first or second medical devices 124. In some embodiments, the analysis module 342 can use artificial intelligence, machine learning, and/or other data analytics to enhance diagnostics, such as to detect and/or reject arrhythmias, such as atrial fibrillation (AF) and/or ventricular fibrillation (VF), track heart failure decompensation, and the like. In other embodiments, when the analysis module 342 identifies a condition that exceeds boundaries, the analysis module 342 can issue an alert to the patient 108, the clinician 112, the remote care app 350, and/or any other designated recipient, such as the vender.

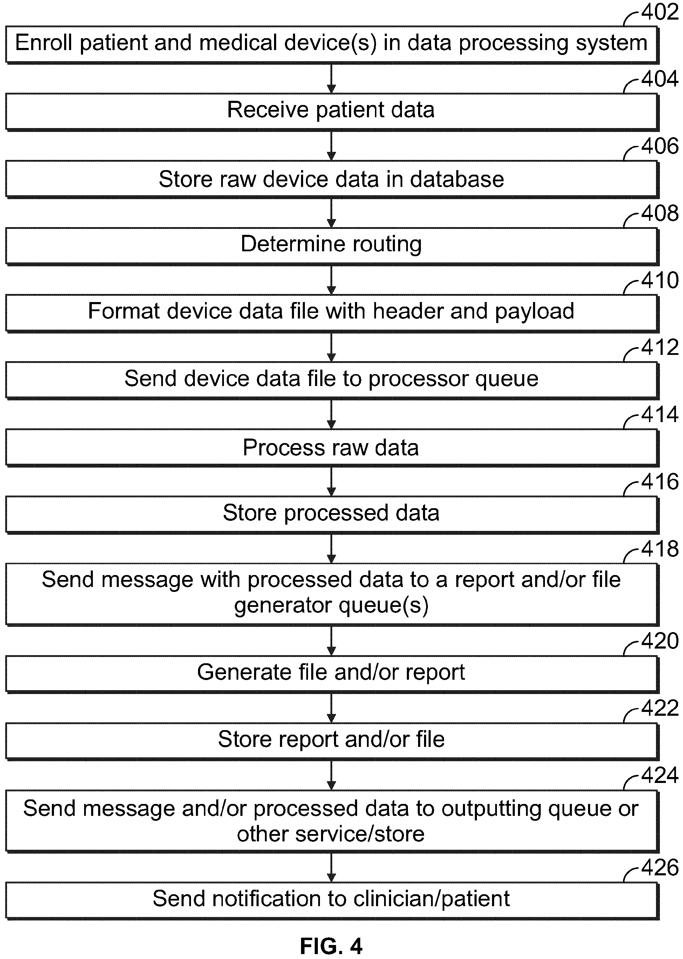

Figure 4 illustrates the process flow of the data processing system 122 for receiving patient data, routing the patient data, processing the patient data and outputting the results in accordance with embodiments herein. The operations of Figure 4 may be implemented by hardware, firmware, circuitry and/or one or more processors housed partially and/or entirely within a local external device, remote server or more generally within a healthcare system, may be distributed, such as throughout a region, and/or may be on different nodes. For example, the external devices/systems (e.g., local, remote or anywhere within the healthcare system 100 or data processing system 122) can include memory and one or more processors. Figures 3 and 4 will be discussed together.

**At** 402, one or more processors of the system 100, regional system 104, and/or data processing system 122 receive enrollment information for the patient 108 and device(s) 124. For example, the patient 108 can have one associated medical device 124 or more than one medical device 124. In some cases, the patient 108 can have two or more medical devices 124 associated with two or more manufacturers. For example, the model number and serial number of each medical device 124 are loaded into the system 100. In some embodiments, the data is entered into the central service center 102 to populate the global device index 120 and the patient 108 is assigned to a regional system 104. In other embodiments, the data processing system 122 can receive, route, and process data without being associated with a global system and/or other regional systems. In some cases, data type (e.g., telemetry type, ECG, CA, pressure) may be included to assist with the routing algorithm, as different medical devices 124 acquire different types of data. At a minimum, enough medical device information is loaded into the system 100 to facilitate the routing and processing of the patient data. For example, if the patient 108 resides in the United States, the medical device information reflects that information and the system 100 will route the patient data to be stored and processed in servers/databases that are approved for use with patient data in the United States.

In some embodiments, the gateway 212 includes a routing cache 336 that is kept based on one or more of device model number, serial number, type of data, software version number, assigned home location of patient 108, etc. Other identifying factors are contemplated. In other cases, the gateway 212 utilizes a routing table, a database, or any other method known in the art for identifying the patient data and correlating the patient data with an identified routing. In further embodiments, the gateway 212 includes software capable of determining system capacities to effectively route the input data in the most efficient manner. In some embodiments, a memory within the system 122 is configured to store the routing cache 336 based on device model numbers or type of medical data to be processed. The routing cache 336 can be configured to identify which of the first or second DDT 214 processes the associated patient data, and the unique ID can be compared to the routing cache.

In some embodiments, if the identifying information loaded in the system 100, data processing system 122, and/or gateway 212 does not match the data received from a medical device 124, the transmitted data may be rejected and an error set. In some cases, the transmitted data may be stored for a period of time or routed to the central service center 102.

At 404, one or more processors of the system 100, regional system 104, and/or data processing system 122 receive, over the internet, POTs, etc., the patient data acquired by the medical device 124 along with device data that identifies the medical device 124. The patient data can include the identifying information as well as raw patient data that has been collected by monitoring physiological signals of the patient, treatment data such as pacing parameters, alert detection parameters (e.g., percent of ventricular pacing threshold, time duration, physician specified thresholds, alert evaluation period), and the like.

**At** 406, one or more processors store the raw data in the data lake 218. Therefore, a copy of the raw data is stored (e.g., archived) in case transmission and/or processing errors occur, corrupting the data. Also, the raw data can be saved for other uses, such as by clinical study application 344, comparison and processing of similar data sets, and by analysis module 342, such as data analytics and/or artificial intelligence.

It should be understood that there may be more than one data lake 218 in the data processing system 122 for storing raw data, and that multiple databases 320 can be stored, for example, in the data lake 218. In some embodiments, the gateway 212 transmits information about the patient medical device 124 to a patient device service 326 and the raw data to a raw data service 328.

At 408, one or more processors, such as of the gateway 212, determine the appropriate routing for the patient data. For example, the gateway 212 identifies the DDT 214 and/or an associated message queue, herein referred to as processor queue 304a, 304b, 304c, such as based on device type and/or model number. For example, the one or more processor determines that the routing of patient data from medical device 124b is to processor queue 304a and the routing of patient data from medical device 124d is to processor queue 304b.

In some embodiments, each of the DDTs 214 is deployed with an associated processor queue 304 (e.g., MQ Server component) to keep each DDT 214 (e.g., processor) as loosely coupled and independent as possible. In some embodiments, the data processing system 122 can include more than one DDT 214 that processes the same type of patient data and/or the data from the same type of medical device 124. For example, each of the DDTs 214 can have an associated processor queue 304, and the gateway 212 can select the processor queue 304 to use based on hierarchy, queue space, etc. In other embodiments, a single processor queue 304 can be configured to "feed" data to more than one DDT 214. In some embodiments, one or more processors are configured to route the patient data to a queue 304 configured to feed an associated one of the first DDT 214 or the second DDT 214, wherein the queue 304 is configured to temporarily store the patient data for the associated DDT 214.

**At** 410, one or more processors format a device data file having a message header that includes request parameters and a message payload that includes raw device data from the medical device 124. In other embodiments, the message received from the medical device 124 can already be in a desired format and may require little or no additional formatting prior to forwarding. Request parameters can include, but are not limited to, the type of transmission, source, device information, preferences, etc.

At 412, one or more processors transmit the device data file to the processor queue 304. In some embodiments, the processor queue 304 can be a first-in-first-out process, while in other embodiments the processor queue 304 may consider hierarchies, such that some data may be indicated as having a higher priority and thus be processed earlier than lower priority data.

If the patient data was received from medical device 124b and routed to processor queue 304a, when the message reaches the top of the queue 304a and the associated DDT 214a is ready to process more data, at 414 the one or more processors receive the device data file from the processor queue 304 and processes the raw data. In some embodiments, the one or more processors extract the request parameters from the message header and the data from the message payload. Alternatively or additionally, if the medical data is not received through a payload, the one or more processors can request the raw data from the data lake 218, such as by using a data service 324.

At 416, when the processing is complete, the one or more processors of the DDT 214 save the processed data in the data lake 218, such as by using the device data service 330.

**At** 418, the one or more processors send (e.g., route) a message with the processed data to a report and/or file generator queue 306a-306d that is configured to temporarily store the processed data for the associated one of the report generator 308 or the file generator 310. The processed data can include transmissions, episodes, reports, alerts, etc. In some embodiments there may be more than one report and/or file generator queue 306 feeding each file generator 310 and/or report generator 308. For example, there may be multiple report generators 308 configured to generate the same types of reports that are fed by a lesser number of queues 306. In other embodiments, one or more of the report and/or file generator queues 306 can have a one-to-one relationship with one report generator 308 or file generator 310.

In some embodiments, the gateway 212 controls the flow of data between and/or through the processor queues 304, the DDTs 214, the report and/or file generator queues 306, and the report and file generators 308, 310. The gateway 212 determines where to send (e.g., route) the particular type of data, as well as monitoring the processing capacity and throughput of each of the devices/instances.

At 420, one or more processors, such as of the report generator 308 and/or file generator 310, receive the message from the report and/or file generator queue 306 and generates the desired output. For example, the report generator 308 can generate a PDF or other graphic file displaying the desired information. Other report formats are contemplated. The file generators 310a, 310b, 310c can generate different types of files. For example, the file generator 310a can collect structured data to use in graphical user interface (e.g., GDF), file generator 310b outputs IEEE format, while file generator 310c outputs PACEART standard format data.

In still further embodiments, one or more processors create a unique virtual programmer process to translate each incoming device data file. In some embodiments, there is a one-to-one association between each incoming device data file and each virtual programmer process, thus parallelly processing multiple device data files.

At 422, one or more processors store reports and/or files in the data lake 218, such as by using the data file service 332. In some embodiments, the one or more processors send at least a portion of the data to a virtual device engine (VDE) 360, and the VDE 360 can store the data in at least one database.

At 424, one or more processors of the report generator 308 and/or file generator 310 output a message (e.g., files, EHR report, other processed data, alerts, percentage ventricular pacing alert, atrial fibrillation alert, recommendations for setting changes and/or behavior modification, trended data) to an outputting queue 312. Although a single outputting queue 312 is shown, it should be understood that based on system requirements and volume, multiple queues 312 may be used and may be dedicated, in some cases, to particular destinations, such as one or more FTP server. The outputting queue 312 can feed the remote care application 350 and/or the HRS 340, and/or another data lake, data store, etc., that may be directly accessible by clients or capable of processing queries from clients. In general, the HRS 340 can be a third party platform that facilitates the exchange of data between different groups. The remote care application 350 can be a system used by a vender that is configured to receive all or select portions of the vender's patient's data. It should be understood that there can be multiple remote care applications 350, wherein each one is associated with a different vender, product, product line, etc. Furthermore, although only a single HRS 340 is shown, the data storage system can be distributed and include many different data storage options (e.g., data receivers 346), and thus particular data may be routed accordingly.

Alternatively and/or optionally, at 426, one or more processors, such as of a remote care application 350 and/or HRS 340, process the message from the outputting queue 312 or other input and transmits a notification to the clinician application 110, patient application 106, and/or other designated address. In some cases, the data processing system 122 sends a message that advises that an EHR report and/or processed data are available. In some embodiments, the data processing system 122 can automatically push the EHR report and/or the processed data to the clinician application 110. In still other embodiments, the data processing system 122 can push a portion of the available data, such as the EHR report, to the clinician application 110, and the clinician application, clinician 112, etc., can choose to access or request the processed data.

Alternatively and/or optionally, the data stored in the data lake 218 can be accessed by a client submitting an API call. One or more processors associated with, for example, a business service 334 and/or data service 324, process the API call, packages the requested data, and transmits the data to the HRS 340, directly to the client, such as the remote care application 350, etc.

The clinical study application 344 is an example of an application system that receives output from the business services 334, such as for use during clinical trials, etc. The appropriate report generator 308 and/or file generator 310 can be configured to push data to the clinical study application 344. In some embodiments, data associated with identified patients 108 may be sent to the clinical study application 344, while in other embodiments, data associated with identified medical device(s) 124 can be sent to the clinical study application 344. The integrated manner of the healthcare system 100 provides the ability to leverage data globally for studies, and may also provide additional data such as location, demographic data, etc.

It should be understood that other business services 334 can be implemented to handle API requests from clients in order to leverage data services 324 and retrieve data from the data lake 218, providing data to the client. In some embodiments, a business application such as the remote care application 350 can be an FTP server that receives uploaded data. The client or customer can then retrieve the data from the FTP server.

In some embodiments, one or more processors can establish a process timeout period to ensure reclamation of the process resources if the process becomes a zombie or does not complete within the allotted time. If the one or more processors determine that a positive completion status is returned in time, the completion status, associated with a transaction ID, can be saved, such as to an administration database in the data lake 218. If a positive completion status is not returned in time, e.g., a process failure is returned or timeout period has expired, a process error handling protocol can be executed based upon the failure type. One type of failure can be a device data file access or content error. An example is an error opening the device data file due to corruption of its contents. Another type of failure can be a device data file processing error. An example is a data collection problem or incompatibility between the collected device data and the data translation process. Other types of failures can be, but are not limited to, a resource error or an out of memory error. Error handling can ensure that there is no missing data and report any issues that occur during data processing after re-trying the processing.

### Data processing system is modular and scalable

**An** advantage of the data processing system 122 is the modularity and scalability of the system 122, e.g., plug-in, plug-out. For example, to add the capability of receiving data from a new type of medical device 124, the appropriate number of queues 304 and DDTs 214 can be added to process the data, and the gateway 212 can pair the new data processor(s) (e.g., DDTs 214) with the new medical device type. When a medical device 124 is removed from service or the system 122 is no longer acquiring the data, the DDT(s) 214 can be removed. Also, multiple DDTs 214 that process the same type of data, such as from the same type of medical device 124 and/or from medical devices 124 from different venders, can be included. The capacity to process data from the same type of medical device 124 can be increased or decreased by adding or removing, respectively, DDTs 214 and/or processing queues 304.

Similarly, a new type of business service 334 can be added by adding new report generator(s) 308 and/or file generator(s) 310, as well as the desired number of queue(s) 306. Services to manage the flow of data in the data services 324 similarly can be replicated, added, and removed.

The data lake 218 is similarly modular and scalable. Additional databases 320 can be added to the network or removed based on capacity, business requirements, and the like.

### Data Processor Server

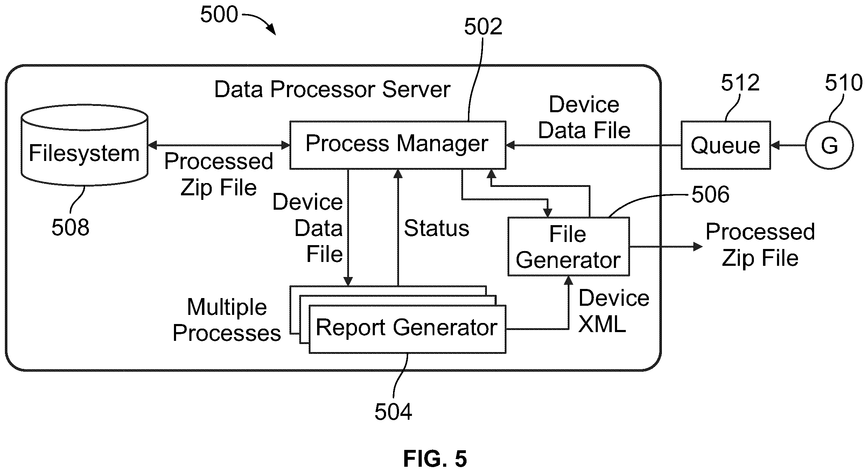

Figure 5 illustrates an exemplary data processor server 500 that is configured to generate reports and/or files from a particular type of medical device 124 and/or a particular type of data in accordance with embodiments herein. Therefore, in some embodiments at least some processes / functionality associated with a particular medical device 124 and/or particular type of data can be modularly grouped together. In the example of Figure 5, the raw data file can be a device data file associated with any type of medical device 124, and is not limited to a specific medical device 124.

Gateway 510 can receive medical and device data, identify the correct data processor server 500 to process the data, and route the data to one or more appropriate queue 512. In some embodiments, the data processor server 500 can process files received from patient applications 106, clinician applications 110, programmers and other communication devices 202, etc., such as via internet data transmissions. These may include manual sneakernet uploads via programmers or other interfaces or communication devices 202. Some transmissions can include one or more of stored EGM data, freeze capture data, trended data, impedance monitoring information, etc.

In some embodiments, the software of the process manager 502 is a generic component installed on the DDTs 214 and/or other data processor servers 500 within the data processing system 122, adding to the flexibility of the system 122. The process manager 502 is configurable at initialization to meet the unique requirements of the data processor server 500 in which it executes. The process manager 502 launches and manages processes to translate raw device data into meaningful data (e.g., in XML and JSON format) although other formats can be used based on the data, end user, desired reports, etc. The process manager 502 generates the follow-up PDF report files (other formats can be used) from which the final reports provided to the clinician 112 will be generated. The process manager 502 also ensures that the processed file is created for routing to downstream components and processes as discussed herein.

In some embodiments, the data processor server 500 can include one or both categories of device data processes, namely report generators 504 (e.g., one or more processors) and file generators 506 (e.g., one or more processors), discussed further herein. The report generators 504, in this context, translate device data into other formats (e.g., XML and JSON format) and generate follow-up PDF reports similar to those output by the programmers used by the clinicians 112. In other words, the follow-up PDF reports can provide substantially similar data, and may be formatted in a substantially similar way, as the reports clinicians 112 and other health care professionals are currently familiar with.

In general, the file generators 506 convert intermediate device data from one format to another for one or more specific purpose. In some embodiments, the file generator 506 converts intermediate device data in XML format into a format which is readily used for other specific purposes such as generation of the IEEE file for export to, for example, an electronic health record system (not shown).

For example, the process manager configuration can be an XML based file which is simple to parse in Java as opposed to a variable length string-based configuration file. This configuration file can include information such as: i) type of medical device 124 or type of data, ii) inter-process communication type (Java or C/C+), iii) error timeout values, iv) error retries attempts upon detected errors, v) associated error mitigation type, vi) incoming device record file directory, vii) outgoing processed data file directory, viii) archive file directories to store all incoming and outgoing files, and ix) other detail design related configuration items.

The data processor server 500 can include or communicate with working and archive directories stored in one or more filesystem 508, database, or other memory, that stores all significant files used and generated during processing. The filesystem 508 may be remote from, and not dedicated solely to, the data processor server 500. Although other directories may be used, the DDT 214 can include working directories such as: i) configuration directory to store server configuration data files, ii) incoming directory to provide a landing directory for incoming files, iii) in-process directory to hold incoming tarballs being processed, and iv) outgoing directory to hold processed files. The data processor server 500 can also include archive directories such as: i) incoming directory to save incoming transmission files, ii) intermediate directory to save all report generator 504 and file generator 506 generated files, iii) failed directory to save incoming tarballs which failed processing; and iv) outgoing directory to save processed files.

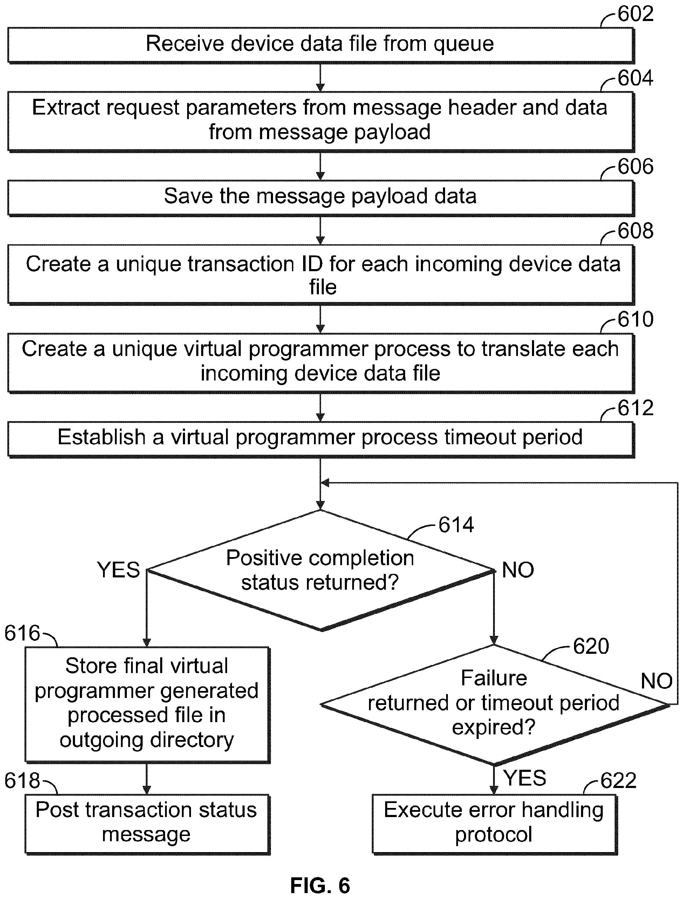

Figure 6 illustrates the process flow for receiving and processing the device data file and outputting the results in accordance with embodiments herein. The operations of Figure 6 may be implemented by hardware, firmware, circuitry and/or one or more processors housed partially and/or entirely within a local external device, remote server or more generally within a healthcare system. For example, the devices/systems (e.g., local, remote or anywhere within the healthcare system 100 or data processing system 122) can include memory and one or more processors. Figures 5 and 6 will be discussed together.

At 602, one or more processors, such as of the process manager 502, receive the device data file from the queue 512. In other embodiments, the process manager 502 or other service retrieves the device data file from the queue 512, or can be directed to retrieve medical data stored in the data lake 218.

At 604, one or more processors extract the request parameters from the message header and the data from the message payload.

At 606, one or more processors save the message payload data as a file into an incoming directory of the data processor server 500. In some embodiments the filename may be specified in the message header.

In some embodiments, at 608, one or more processors create a unique transaction ID for each incoming device data file to monitor the processing. In some cases, within the data processing system 122, the data processor servers 500 can maintain all transaction log information in an administration database. This log information can be used to search for a transaction in addition to gleaning historical transaction statistical data.

At 610, one or more processors create a unique virtual programmer process to translate each incoming device data file. In some embodiments, there is a one-to-one association between each incoming device data file and each virtual programmer process (e.g., a different report generator process for each device data file). Therefore, the following processes can be run in parallel to parallelly process multiple device data files.

At 612, one or more processors establish a virtual programmer process timeout period to ensure reclamation of the process resources if the process becomes a zombie or does not complete within the allotted time.

At 614, one or more processors determine if the report generator(s) 504 have returned a positive completion status. In some embodiments, the process manager 502 saves the completion status, associated with the transaction ID, to the administration database.

If a positive completion status is returned, flow passes to 616 and one or more processors store the final virtual programmer generated processed file in an outgoing directory.

At 618, one or more processors post a transaction status message to confirm that processing of the transmission has been completed.

Returning to 614, if a positive completion status is not returned, flow passes to 620 and the one or more processors determine if a virtual programmer process failure is returned or timeout period has expired. If no, flow returns to 614 to monitor for positive completion status.

If yes at 620, at 622 one or more processors execute the process error handling protocol based upon the failure type. One type of failure can be a device data file access or content error. An example is an error opening the device data file due to corruption of its contents. Another type of failure can be a device data file processing error. An example is a data collection problem or incompatibility between the collected device data and the data translation process. Yet another type of failure can be a virtual programmer process resource error. One example is an out of heap space memory error.

In some embodiments, at regular intervals and/or on demand, the one or more processors, such as of the process manager 502, can save report generator 504 (e.g., virtual programmer) process status and performance information to the administration database. Some examples of the report generator processing status include: i) duration of device data processing (minimum, maximum, and average), ii) results of device data processing, and/or iii) minimum, average, and maximum size of the processed tarball file.

The file generator 506 can receive data from the process manager 502 and/or data from the report generator 504. The process manager 502 can also monitor the file generator 506 for errors.

### Report Generator

In some embodiments, the report generator 504 (e.g., software component) translates device data files into a master data file (MDF) stored in XML format. Device follow-up data, such as clinical parameters, episodes, diagnostics, etc., can be translated into this XML format. The MDF is the key output product of the virtual programmer, allowing simpler manipulation of the device data to generate other specific reports and allowing importation into one or more database.

The report generator 504 can work together with one or more other components/instances/modules/processors to generate follow-up reports, files, and the like, based on the type of data processed, requests included in the device data file (e.g., parameters set by the health care professional), etc. The report generator 504 can be programmed to generate the files in a number of formats, such as Postscript format and/or PDF. The virtual programmer generated intermediate zip file can contain one or more of the master data file, PDF reports, Paceart file, and the original raw device data files. This intermediate file can be utilized by the file generator 506 to create a set of specific data files, such as those to be sent to the remote care application 350.

For example, for a data processor server 500 that processes ICM data, the report generator 504 can process the ECG data from patient app session record(s). The output of this processing can be a JSON file that contains the waveform and marker data for each episode.

### File Generator

The file generator 506 can generate a GUI Data File (GDF). This object-oriented component is highly configurable and generic enough to recognize different ones of the intermediate file formats to parse.

The function of the file generator 506 is specific to the data processor server 500 in which in resides. By designing a generic software component, the specific functionality the file generator 506 provides on the data processor server 500 is determined by the file generator 506 configuration file read at initialization. The file generator 506 is a multi-threaded design to accommodate the many incoming transmissions in the data processor server 500. For example, the contents of the processed data file output by each of the specific data processor servers 500 varies based on the device type and/or data type.

### IMD

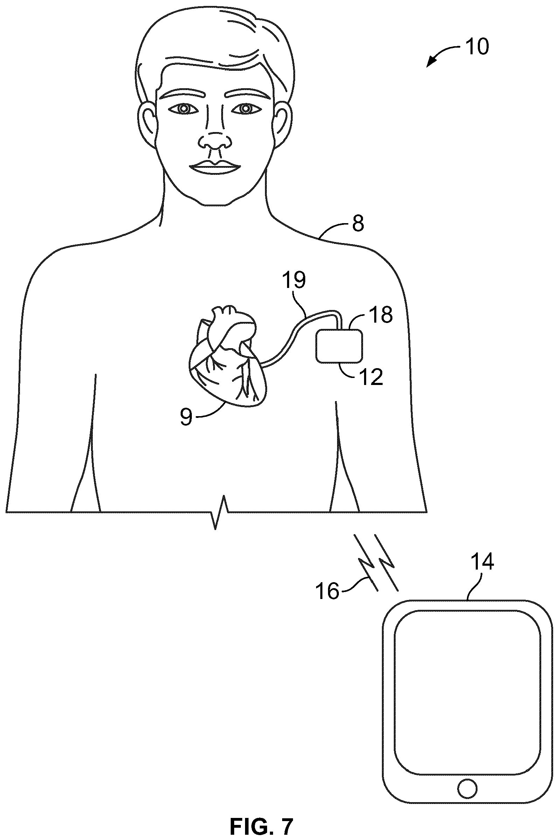

Figure 7 illustrates a simplified block diagram of a system 10 operated in accordance with embodiments herein. The system 10 includes an IMD 12 (e.g., medical device 124) and one or more external devices or instruments. A single external device 14 (e.g., communication device 202) is illustrated in Figure 7. The IMD 12 and the external device 14 are configured to communicate with one another wirelessly over a wireless communication link 16. In an embodiment, the external device 14 receives communications from the data processing system 122 and transmits communications, including physiological data acquired from the patient 108 and one or more of information identifying the IMD 12 and the type of data acquired by the IMD 12, to the data processing system 122.

The IMD 12 is implanted within a patient 8 at a site near the heart 9. The IMD 12 may be a cardiac pacemaker, an implantable cardiac monitoring device (ICM), a defibrillator, an ICM coupled with a pacemaker, or the like. The IMD 12 is intended for implantation within a subcutaneous pocket of the patient. The IMD 12 may be configured to sense cardiac signals to monitor cardiac activity over time. Optionally, the IMD 12 may also be configured to deliver stimulation therapy to the heart 9. For example, the IMD 12 may be a dual-chamber stimulation device capable of treating both fast and slow arrhythmias with stimulation therapy, including cardioversion, defibrillation, and pacing stimulation, as well as capable of detecting heart failure, evaluating its severity, tracking the progression thereof, and controlling the delivery of therapy and warnings in response thereto.

The IMD 12 in the illustrated embodiment includes a body or housing 18 that is connected to at least one lead 19. A single lead 19 is shown in Figure 7, but the IMD 12 may include multiple leads in another embodiment. The lead 19 extends from the housing 18 to the heart 9, such that the distal end is in contact with patient tissue surrounding the heart 9. The lead 19 includes one or more electrodes that may measure cardiac signals of the heart 9 and deliver stimulation therapy to the heart 9. In an embodiment, a single electrode may emit a stimulation pulse in a stimulation mode, and then may quickly switch to a monitoring mode to detect cardiac signals following the stimulation pulse.

Although the IMD 12 in the illustrated embodiment includes a lead 19, one or more of the embodiments described herein utilize a leadless IMD that does not include any lead. For example, the IMD 12 may be a leadless pacemaker, a leadless cardiac monitoring device (ICM), or the like. In general, the IMD 12 may represent a cardiac monitoring device, pacemaker, cardioverter, cardiac rhythm management device, defibrillator, neurostimulator, leadless monitoring device, leadless pacemaker, and the like.

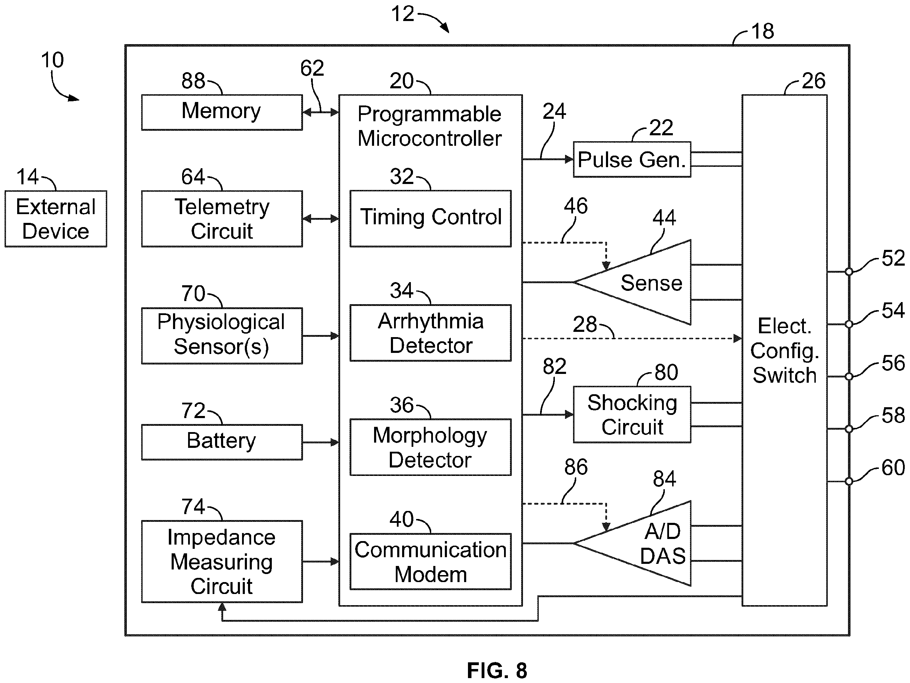

The housing 18 may contain a battery, pulse generation circuitry, communication circuitry, a data storage device (e.g., memory), and/or control circuitry including one or more processors. The control circuitry is for receiving and analyzing electrocardiogram IEGM signals from the electrodes. The control circuitry may include at least one processor for processing the IEGM signals in accordance with algorithms to make determinations about the state of the heart 9. The memory provides storage for the cardiac signals and programmed instructions for the control circuitry that provide a programming session. The battery powers the circuitry with the housing 18. For example, the battery powers the pulse generation circuitry to generate stimulation pulses and powers the communication circuitry to communicate with an external device 14. The control circuitry may generate messages to be communicated via the communication circuitry to the external device 14. The messages may include the IEGM signals and/or data generated based on the IEGM signals. The control circuitry is also configured to analyze messages, received via the communication circuit from the external device 14, that include programming packages for updating the operating configuration, including settings, parameters, behavior, and the like, of the IMD 12. Exemplary structure for the IMD 12 is discussed and illustrated below in connection with Figure 8.

The external device 14 may represent a computing device that is accessible or possessable by the patient or a clinician, such as a tablet computer, a smartphone, a wearable device, laptop computer, a desktop computer, a bedside monitor installed in a patient's home, or the like. The external device 14 alternatively may be a programmer device used in the clinic by a clinician. The external device may be one of the devices listed above that is communicatively connected to a second external device that represents the source of the programming package intended to update the operating configuration of the IMD 12. For example, the second external device may be a server or another computing device that is communicatively connected to the first external device 14 via a network connection (e.g., the Internet). In general, the external device 14 facilitates access by physicians to patient data as well as permits the physician to review real-time electrocardiogram (ECG) signals and, as specifically described herein, update the operating configuration of the IMD 12 without the clinician being near the patient.

Figure 8 shows a block diagram of the system 10 in accordance with embodiments herein. The housing 18 or case of the IMD 12 holds the electronic and/or computing components. The housing 18 further includes a connector (not shown) with at least one terminal 52 and optionally additional terminals 54, 56, 58, 60. The terminals may be connected to electrodes that are located in various locations within and about the heart, such as on the lead 19 (shown in Figure 7). The electrodes may include various combinations of ring, tip, coil, shocking electrodes, and the like.

The IMD 12 includes a programmable microcontroller 20 that controls various operations of the IMD 12, including cardiac monitoring and stimulation therapy. Microcontroller 20 includes one or more processors (e.g., a microprocessor or equivalent control circuitry), RAM and/or ROM memory, logic and timing circuitry, state machine circuitry, and I/O circuitry. Microcontroller 20 includes an arrhythmia detector 34 that is configured to detect cardiac activity data to identify potential atrial fibrillation (AF) episodes as well as other arrhythmias (e.g., Tachycardias, Bradycardias, Asystole, etc.).

An electrode configuration switch 26 is optionally provided to allow selection of different electrode configurations under the control of the microcontroller 20. The electrode configuration switch 26 may include multiple switches for connecting the desired electrodes to the appropriate I/O circuits, thereby facilitating electrode programmability. The switch 26 is controlled by a control signal 28 from the microcontroller 20. Optionally, the switch 26 may be omitted and the I/O circuits directly connected to a housing electrode.

The IMD 12 may include a chamber pulse generator 22 that generates stimulation pulses for connecting the desired electrodes to the appropriate I/O circuits, thereby facilitating electrode programmability. The pulse generator 22 is controlled by the microcontroller 20 via control signals 24. The IMD 12 includes a sensing circuit 44 selectively coupled to one or more electrodes that perform sensing operations through the switch 26 to detect cardiac activity. The sensing circuit 44 may include dedicated sense amplifiers, multiplexed amplifiers, or shared amplifiers. The sensing circuit 44 may operate in a unipolar sensing configuration or a bipolar sensing configuration. The output of the sensing circuit 44 is connected to the microcontroller 20 which, in turn, triggers, or inhibits the pulse generator 22 in response to the absence or presence of cardiac activity. The sensing circuit 44 receives a control signal 46 from the microcontroller 20 for purposes of controlling the gain, threshold, polarization, and timing of any blocking circuitry (not shown) coupled to the sensing circuit.

The IMD 12 further includes an analog-to-digital A/D data acquisition system (DAS) 84 coupled to one or more electrodes via the switch 26 to sample cardiac signals across any pair of desired electrodes. The A/D DAS 84 is controlled by a control signal 86 from the microcontroller 20.

The IMD 12 is further equipped with a communication circuit or modem (modulator/demodulator) 40 to enable wireless communication. The modem 240 enables timely and accurate data transfer directly from the patient to the external device 14, and vice-versa. For example, the communication modem 40 is configured to establish the communication link 16 (Figure 7) with the external device 14. In an embodiment, the communication modem 40 can receive a programming package from the external device 14 and conveys the programming package for updating, changing, varying, etc., the programming session to the microcontroller 20 for verification prior to executing configuration change requests and/or command execution requests contained within the programming package. In addition to remote programming of the IMD 12, the wireless communication link 16 with the external device 14 facilitates access by physicians and/or patients to patient data generated by the IMD 12.

The communication modem 40 may utilize radio frequency (RF), Bluetooth, or Bluetooth Low Energy telemetry protocols. The signals are transmitted in a high frequency range and will travel through the body tissue in fluids without stimulating the heart or being felt by the patient. The communication modem 40 may be implemented in hardware as part of the microcontroller 20, or as software/firmware instructions programmed into and executed by the microcontroller 20. Alternatively, the modem 40 may reside separately from the microcontroller 20 as a standalone hardware component.

The microcontroller 20 is coupled to a non-transitory data storage device, referred to herein as memory device 88, by a suitable data/address bus 62. The memory device 88 stores programmable operating parameters used by the microcontroller 20 and/or data associated with the detection and determination of arrhythmias. In an embodiment, the memory device 88 also stores the current programming session, along with any and all changes, modifications, updates, or the like previously made to the programming session.

The IMD 12 optionally includes one or more physiologic sensors 70 that adjust pacing stimulation rates, detect changes in cardiac output, changes in the physiological condition of the heart, and/or diurnal changes in activity (e.g., detecting sleep and wake states). Examples of physiological sensors 70 might include sensors that, for example, sense respiration rate, pH of blood, ventricular gradient, activity, body movement, position/posture, minute ventilation (MV), and/or the like.

The battery 72 provides operating power to all of the components in the IMD 12. The battery 72 is capable of operating at low current drains for long periods of time, and is capable of providing high-current pulses (for capacitor charging) when the patient requires a shock pulse (e.g., in excess of 2 A, at voltages above 2 V, for periods of 10 seconds or more).

The IMD 12 further includes an impedance measuring circuit 74, which can be used for many things, including sensing respiration phase. The IMD 12 may be further equipped with a telemetry circuit 64 that can selectively communicate with an external device, such as the device 14, when connected via a physical (e.g., wired) communication link. The IMD 12 includes a shocking circuit 80 controlled by control signals 82 generated by the microcontroller 20. The shocking circuit 80 generates shocking pulses of low (e.g., up to 0.5 joules), moderate (e.g., 0.5-10 joules), or high energy (e.g., 11 to 40 joules), as controlled by the microcontroller 20. In an alternative embodiment in which the IMD 12 senses and monitors cardiac activity without administering stimulation therapy, the IMD 12 may lack the pulse generator 22 and the shocking circuit 80.

The microcontroller 20 may include other dedicated circuitry and/or firmware/software components, such as a timing control (module) 32 and a morphology detector (module) 36. The timing control 32 is used to control various timing parameters, such as stimulation pulses (e.g., pacing rate, atria-ventricular (AV) delay, atrial interconduction (A-A) delay, ventricular interconduction (V-V) delay, etc.) as well as to keep track of the timing of RR-intervals, refractory periods, blanking intervals, noise detection windows, evoked response windows, alert intervals, marker channel timing, and the like. The morphology detector 36 is configured to review and analyze one or more features of the morphology of cardiac activity signals, such as the morphology of detected R waves to determine whether to include or exclude one or more beats from further analysis.

### Healthcare System

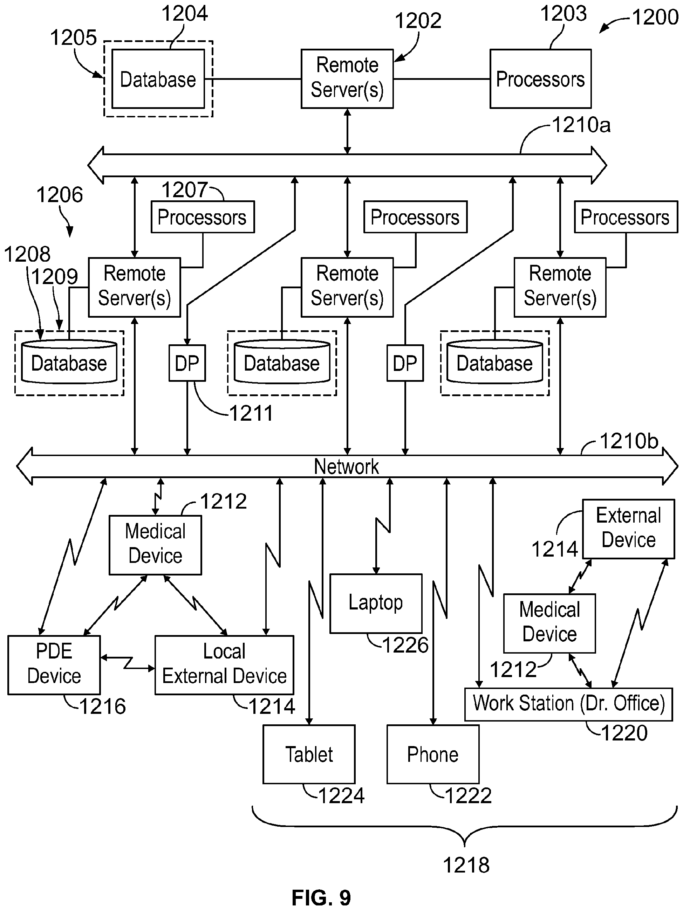

Figure 9 illustrates a healthcare system 1200 interfacing with a plurality of networked devices formed in accordance with embodiments herein. The healthcare system 1200 includes one or more servers 1202 and one or more processors 1203 connected to one or more database 1204 that is stored in one or more memory 1205, similar to the central service center 102 of Figure 1. The healthcare system 1200 further includes one or more servers 1206 and one or more processors 1207 connected to one or more database 1208 that is stored in one or more memory 1209, and one or more data processing system 1211 similar to the regional systems 104 of Figure 1, wherein each of the regional systems 104 can include one or more servers 1206, one or more processors 1207, one or more data processing system 1211, and one or more database 1208 stored in one or more memory 1209. The servers 1206 and databases 1208 within a regional system 104, as well as the servers 1202 and databases 1204 of the central service center 102, can be located at a common physical location and/or distributed between multiple remote locations within a city, state, country, region, and/or the world. The one or more servers 1206 and one or more databases 1208 of different regional systems 104 can be located in different regions.

The server(s) 1202 communicate with one or more network 1210a, and the servers 1206 communicate with one or more networks 1210a, 1210b. The network 1210 may be a private network, the internet, a voice over IP (VoIP) gateway, a local plain old telephone service (POTS) such as a public switched telephone network (PSTN), a cellular phone-based network, a combination of one or more networks, and the like. Alternatively, the network 1210 may be a local area network (LAN), a campus area network (CAN), a metropolitan area network (MAN), or a wide area network (WAM). Although shown separately, the networks 1210a and 1210b can be connected to each other, such as to the internet. The network 1210b serves to provide a network that facilitates the transfer/receipt of information such as medical data, medical device data, patient data, data type, communications between medical devices 124, communication devices 202, and the data processing system 122, and patient data such as PHI and PII.

The system 1200 also includes one or more medical devices 1212 (e.g. medical devices 124), one or more local external devices 1214 (e.g., communication devices 202), one or more patient data entry (PDE) devices 1216 and one or more medical personnel (MP) devices 1218, all of which communicate (directly or indirectly) through the network 1210b to the servers 1206 and/or one another. The medical device 1212 may be passive or active, may collect various types of data, such as cardiac electrical and/or mechanical activity data, PAP or other pressure related data, impedance data, RPM data, flow data, be an IMD, pressure sensor, and the like. The PDE device 1216 collects data, such as based on manual inputs from a patient or other user, and/or based on automatic monitoring devices, such as the medical devices 1212 and/or local external devices 1214.

The local external device 1214 may be implemented as a variety of non-implantable devices including, but not limited to, a medical monitoring and/or medical services delivery device, BGA test devices, medical personnel programmer, a local RF transceiver and a user workstation, smart phone, tablet device, laptop computer, desktop computer and the like.

Accordingly, the medical devices 1212 and external devices 1214 deliver a particular treatment for a medical condition such as pacing, shocking, and the like for arrhythmia (e.g., VA, VT, AF, etc.) and/or other heart conditions, modifications to device settings and parameters, treatment of BGA conditions based on measurements of BGA test devices, providing patient education and behavior modification suggestions, and the like. Further, the medical devices 1212 and/or external devices 1214 deliver the particular treatment which transforms, such as in the case of arrhythmia, the patient's heart from an arrhythmia state to a normal sinus rhythm state.

The MP devices 1218 may also be implemented as a variety of devices including, but not limited to, medical personnel programmer, workstation 1220, smart phone 1222, tablet device 1224, laptop computer 1226, desktop computer and the like. Functionality of the MP devices 1218 related to embodiments herein may be implemented through dedicated hardware circuits, firmware, software, and/or application operating on one or more computing devices.

The server 1202 (e.g., central service center 102) controls the communication of information and requests between the servers 1206 including, but not limited to, at least some of patient data controlled by PDP requirements in one or more regions, IMD data, patient entered data, medical personnel entered data, such as to add a medical device 1212, move a medical device 1212 to or from another region, activate or inactivate a medical device 1212, and/or record information.

For example, in some embodiments, a healthcare system comprises a central service center 102 having memory 1205 and one or more processors 1203. The memory 1205 is configured to store program instructions and a global device index 120 that includes database records associated with medical devices 1212. Each of the records includes a corresponding unique device identifier (ID), region code 210 and status indicator. The region code 210 is associated with a region having corresponding patient data privacy (PDP) requirements, and the status indicator is indicative of whether the corresponding medical device 1212 is active in the corresponding region. The one or more processors 1203, when executing the program instructions, are configured to at least one of: i) manage transfer of patient data, for a first medical device 1212, between first and second regional systems 104 based on the PDP requirements for the corresponding first and second regional systems 104; ii) when the first and second regional systems 104 maintain corresponding status indicators associated with a common medical device 1212, communicate with the first and second regional systems 104 to manage the status indicators, maintained by the first and second regional systems 104, associated with the common medical device 1212; iii) responsive to a registration request, for a candidate device, from the first regional system 104, return a registration response indicating either: a) the candidate device is registered in another regional system 104 or b) the candidate device is available for registration; or iv) responsive to an un-registration request, for a registered device, from the first regional system 104, update the status indicator in the global device index 120 for the registered device in connection with the first regional system 104. In some embodiments, the unique ID includes at least one of i) a model number, ii) a serial number, iii) a unique key, iv) a second unique key, v) a medical device type, vi) birthdate, or vii) type of medical data to be processed. In other embodiments, the global device index 120 is further configured to store, associated with the unique IDs, data that does not include patient information identified by the PDP requirements of the first and second regional systems 104.

Other servers and memory systems (not shown) can receive data from the devices for storage, retrieval, data collection, data analysis, diagnosis, treatment recommendations and the like. Other databases and file structures (not shown) store all or various portions of the information described herein, including, but not limited to, IMD data, medical record information, treatment diagnoses and recommendations, and the like. The local external device 1214 may reside in a patient's home, a hospital, or a physician's office, or be interconnected to a patient's skin surface. The local external device 1214 communicates wired or wirelessly with one or more medical device 1212 and/or the network 1210b. The servers and their associated devices described herein may wirelessly communicate with one another utilizing various protocols, such as Bluetooth, GSM, infrared wireless LANs, HIPERLAN, 3G, satellite, as well as circuit and packet data protocols, and the like. Alternatively, a hard-wired connection may be used to connect the servers and devices. The local external device 1214, when implemented as a programmer, may be configured to acquire cardiac signals from the surface of a person (e.g., ECGs), and/or intra-cardiac electrogram (e.g., IEGM) signals from the medical device 1212. The local external device 1214 interfaces with the network 1210b to upload the data and other information to the appropriate server. Optionally, the local external device 1214 may represent a local RF transceiver that interfaces with the network 1210b to upload IMD data and/or BGA data.

The workstation 1220, in some embodiments found within a doctor or other medical office, may interface with the network 1210b via the internet or POTS to download various data, information, diagnoses, and treatment recommendations from the database 1208. Alternatively, the workstation 1220 may download raw data from the surface ECG units, leads, or monitoring device via either the programmer or the local RF transceiver. Once the user workstation 1220 has downloaded the cardiac signal waveforms, ventricular and atrial heart rates, or detection thresholds, and the like, the user workstation 1220 may process the information. The system may download information and notifications to the smart phone 1222, the tablet device 1224, the laptop computer 1226, or to a server to be stored on a database.

Thus, provided is a distributed "digital" healthcare system that collects various types of data, enables the data to be processed and/or analyzed by various computing devices within the system and securely communicates with clinicians and other healthcare providers, patients and their devices, secure storage solutions (e.g., data lake, database, FTP server). Advantages of the system include but are not limited to modularity and scalability in processing and preparing different types of data and data from different types of devices for consumption by clinicians. Data from medical devices provided by different venders can be collected and processed, providing advantages for accessing and reviewing data by the clinician. For example, a patient may have more than one associated medical device, each provided by a different vender, and all of the data can be collected and processed by the system 1200. Processing capabilities can easily be added and removed to accommodate new medical devices, removal of obsolete medical devices, as well as software revisions and the like, all without negatively impacting other aspects of the system 1200. Thus, an improved system and methodology are provided.

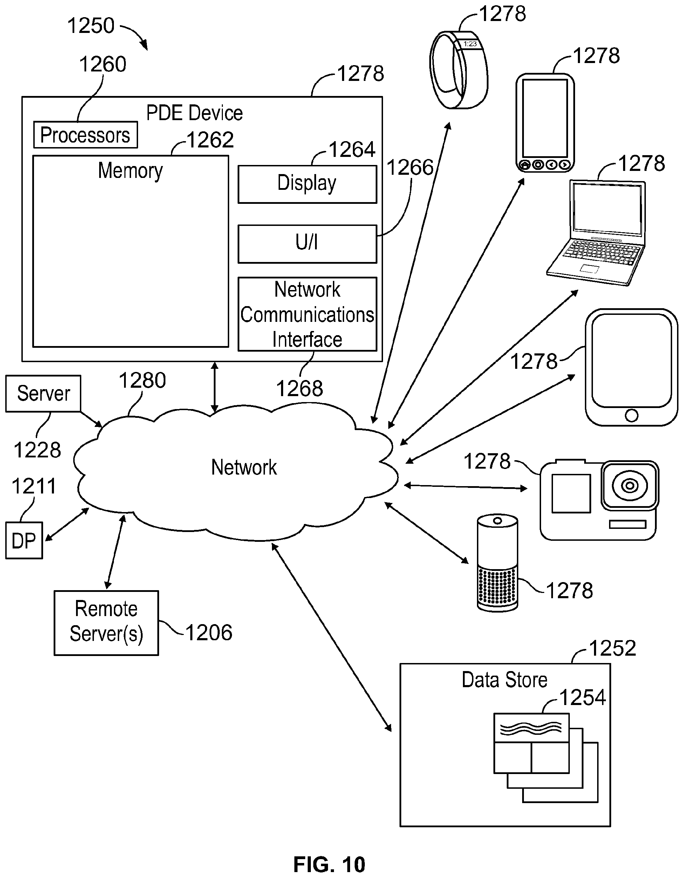

Figure 10 illustrates a distributed healthcare system 1250 that can be included in one of the regional systems 104 in accordance with embodiments herein. The system 1250 can collect and analyze patient data, and communicate over a network 1280 with one or more server 1228 that can in turn communicate with server(s) 1206 and data processing system(s) 122 within the regional system 104. The system 1250 includes one or more patient data entry (PDE) devices 1278 that communicate over the network 1280 with various other devices, such as IMDs, body generated analyte (BGA) test devices, MP devices, local external devices, servers and the like. Optionally, the PDE devices 1278 may communicate through a wholly or partially wired subsystem. The network 1280 may represent the World Wide Web, a local area network, a wide area network and the like. When the PDE device 1278 includes a GUI, the patient or other user may input patient data in addition to IMD data, internal and external medical device data, and BGA data. Optionally, the PDE devices 1278 may include one or more microphones that are configured to listen for audible information spoken by a user or patient, such as a verbal statement to enter patient data. Optionally, the PDE devices 1278 may include one or more cameras that are configured to capture still images and/or video that is processed utilizing image recognition to identify what action a patient is performing (e.g., what, when and how much a patient is eating and/or drinking). The PDE device 1278 includes one or more processors 1260, memory 1262, a display 1264, a user interface 1266, a network communications interface 1268, and various other mechanical components, electrical circuits, hardware and software to support operation of the PDE device 1278. It is recognized that not all PDE devices 1278 include a display, user interface, and the like. For example, a fixed or handheld camera may simply include camera related electronics and network circuitry to support communication to and from the camera.

The user interface 1266 is configured to receive information from a patient or clinician. The user interface 1266 may include a variety of visual, audio, and/or mechanical devices. For example, the user interface 1266 can include a visual input device such as an optical sensor or camera, an audio input device such as a microphone, and a mechanical input device such as a keyboard, keypad, selection hard and/or soft buttons, switch, touchpad, touch screen, icons on a touch screen, touch sensitive areas on a touch sensitive screen and/or any combination thereof. Similarly, the user interface 1266 can include a visual output device such as a liquid crystal display screen, one or more light emitting diode indicators, an audible output device such as a speaker, alarm and/or buzzer, and a mechanical output device such as a vibrating mechanism. The display may be touch sensitive to various types of touch and gestures. As further examples, the user interface 1266 may include a touch sensitive screen, a non-touch sensitive screen, a text-only display, a smart phone display, an audible output (e.g., a speaker or headphone jack), and/or any combination thereof. The user interface 1266 permits the user to select one or more of a switch, button or icon in connection with various operations of the PDE device 1278.

The memory 1262 can encompass one or more memory devices of any of a variety of forms (e.g., read only memory, random access memory, static random-access memory, dynamic random access memory, etc.) and can be used by the processor 1260 to store and retrieve data. The data that is stored by the memory 1262 can include, but need not be limited to, operating systems, applications, and other information. Each operating system includes executable code that controls basic functions of the communication device, such as interaction among the various components, communication with external devices via a wireless transceivers and/or component interface, and storage and retrieval of applications and data to and from the memory 1262. Each application includes executable code that utilizes an operating system to provide more specific functionality for the communication devices, such as file system service and handling of protected and unprotected data stored in the memory 1262.

The network communications interface 1268 provides a direct connection to other devices, auxiliary components, or accessories for additional or enhanced functionality, and in particular, can include a USB, a lightning, or other universal/standard port for linking to a user device with an applicable cable. Optionally, the network communications interface 1268 may include one or more transceivers that utilize a known wireless technology for communication.

The memory 1252 may store object catalogs 1254 organized in various manners and related to a wide variety of objects and types of data. The object catalogs 1254 may be organized and maintained within any manner of data sources, such as data bases, text files, data structures, libraries, relational files, flat files and the like. The object catalogs 1254 include various types of templates corresponding to different types of objects.

### Closing Statements

It should be clearly understood that the various arrangements and processes broadly described and illustrated with respect to the Figures, and/or one or more individual components or elements of such arrangements and/or one or more process operations associated of such processes, can be employed independently from or together with one or more other components, elements and/or process operations described and illustrated herein. Accordingly, while various arrangements and processes are broadly contemplated, described and illustrated herein, it should be understood that they are provided merely in illustrative and non-restrictive fashion, and furthermore can be regarded as but mere examples of possible working environments in which one or more arrangements or processes may function or operate.

As will be appreciated by one skilled in the art, various aspects may be embodied as a system, method or computer (device) program product. Accordingly, aspects may take the form of an entirely hardware embodiment or an embodiment including hardware and software that may all generally be referred to herein as a "circuit," "module" or "system." Furthermore, aspects may take the form of a computer (device) program product embodied in one or more computer (device) readable storage medium(s) having computer (device) readable program code embodied thereon.

Any combination of one or more non-signal computer (device) readable medium(s) may be utilized. The non-signal medium may be a storage medium. A storage medium may be, for example, an electronic, magnetic, optical, electromagnetic, infrared, or semiconductor system, apparatus, or device, or any suitable combination of the foregoing. More specific examples of a storage medium would include the following: a portable computer diskette, a hard disk, a random access memory (RAM), a dynamic random access memory (DRAM), a read-only memory (ROM), an erasable programmable read-only memory (EPROM or Flash memory), a portable compact disc read-only memory (CD-ROM), an optical storage device, a magnetic storage device, or any suitable combination of the foregoing.

Program code for carrying out operations may be written in any combination of one or more programming languages. The program code may execute entirely on a single device, partly on a single device, as a stand-alone software package, partly on single device and partly on another device, or entirely on the other device. In some cases, the devices may be connected through any type of network, including a local area network (LAN) or a wide area network (WAN), or the connection may be made through other devices (for example, through the Internet using an Internet Service Provider) or through a hard wire connection, such as over a USB connection. For example, a server having a first processor, a network interface, and a storage device for storing code may store the program code for carrying out the operations and provide this code through its network interface via a network to a second device having a second processor for execution of the code on the second device.

Aspects are described herein with reference to the Figures, which illustrate example methods, devices and program products according to various example embodiments. These program instructions may be provided to a processor of a general purpose computer, special purpose computer, or other programmable data processing device or information handling device to produce a machine, such that the instructions, which execute via a processor of the device implement the functions/acts specified. The program instructions may also be stored in a device readable medium that can direct a device to function in a particular manner, such that the instructions stored in the device readable medium produce an article of manufacture including instructions which implement the function/act specified. The program instructions may also be loaded onto a device to cause a series of operational steps to be performed on the device to produce a device implemented process such that the instructions which execute on the device provide processes for implementing the functions/acts specified.

The units/modules/applications herein may include any processor-based or microprocessor-based system including systems using microcontrollers, reduced instruction set computers (RISC), application specific integrated circuits (ASICs), field-programmable gate arrays (FPGAs), logic circuits, and any other circuit or processor capable of executing the functions described herein. Additionally or alternatively, the modules/controllers herein may represent circuit modules that may be implemented as hardware with associated instructions (for example, software stored on a tangible and non-transitory computer readable storage medium, such as a computer hard drive, ROM, RAM, or the like) that perform the operations described herein. The above examples are exemplary only and are thus not intended to limit in any way the definition and/or meaning of the term "controller." The units/modules/applications herein may execute a set of instructions that are stored in one or more storage elements, in order to process data. The storage elements may also store data or other information as desired or needed. The storage element may be in the form of an information source or a physical memory element within the modules/controllers herein. The set of instructions may include various commands that instruct the modules/applications herein to perform specific operations such as the methods and processes of the various embodiments of the subject matter described herein. The set of instructions may be in the form of a software program. The software may be in various forms such as system software or application software. Further, the software may be in the form of a collection of separate programs or modules, a program module within a larger program or a portion of a program module. The software also may include modular programming in the form of object-oriented programming. The processing of input data by the processing machine may be in response to user commands, or in response to results of previous processing, or in response to a request made by another processing machine.

It is to be understood that the subject matter described herein is not limited in its application to the details of construction and the arrangement of components set forth in the description herein or illustrated in the drawings hereof. The subject matter described herein is capable of other embodiments and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items.

It is to be understood that the above description is intended to be illustrative, and not restrictive. For example, the above-described embodiments (and/or aspects thereof) may be used in combination with each other. In addition, many modifications may be made to adapt a particular situation or material to the teachings herein without departing from its scope. While the dimensions, types of materials and coatings described herein are intended to define various parameters, they are by no means limiting and are illustrative in nature. Many other embodiments will be apparent to those of skill in the art upon reviewing the above description. The scope of the embodiments should, therefore, be determined with reference to the appended claims, along with the full scope of equivalents to which such claims are entitled. In the appended claims, the terms "including" and "in which" are used as the plain-English equivalents of the respective terms "comprising" and "wherein." Moreover, in the following claims, the terms "first," "second," and "third," etc., are used merely as labels, and are not intended to impose numerical requirements on their objects or order of execution on their acts.

## Claims

1. A healthcare system (100), the healthcare system (100) comprising:
memory configured to store program instructions;
a first device data translator (DDT) (214; 214a) and a second DDT (214; 214b), the first DDT (214; 214a) configured to process data from a first type of medical device (124; 124a), the second DDT (214; 214b) configured to process data from a second type of medical device (124; 124b) that is different from the first type of medical device (124; 124a); and
one or more processors that, when executing the program instructions, are configured to:
receive patient data, the patient data comprising physiological data and at least one corresponding unique device identifier (ID) associated with a first medical device (124), wherein the first medical device (124) is configured to acquire the physiological data from a patient (108);
determine whether the unique ID is associated with the first DDT (214; 214a) or the second DDT (214; 214b); and
route patient data associated with the first medical device (124) to the first DDT (214; 214a) or the second DDT (214; 214b) that is associated with the unique ID.

2. The system of claim 1, wherein the unique ID includes at least one of i) a model number, ii) a serial number, iii) a unique key, iv) a second unique key, v) medical device type, or vi) type of medical data to be processed.

3. The system of claim 1 or 2, wherein the one or more processors are configured to compare the unique ID to a global device index to determine a medical device type or a type of medical data to be processed.

4. The system of any one of claims 1 to 3, wherein
the memory is configured to store a routing cache (336) based on device model numbers or type of medical data to be processed, the routing cache (336) configured to identify which of the first DDT (214; 214a) or second DDT (214; 214b) processes the associated patient data; and
the one or more processors (122) are configured to compare the unique ID to the routing cache.

5. The system of any one of claims 1 to 4, wherein the one or more processors are configured to route the patient data to a queue (304a, 304b) configured to feed an associated one of the first DDT (214; 214a) or the second DDT (214; 214b), wherein the queue (304a, 304b) is configured to temporarily store the patient data for the associated DDT (214; 214a, 214b).

6. The system of any one of claims 1 to 5, wherein the one or more processors are further configured to:
route processed data from the first DDT (214; 214a) to one of a report generator (308) or a file generator (310a-310c); and
route processed data from the second DDT (214; 214b) to one of the report generator (308) or the file generator (31 0a-31 0c).

7. The system of any one of claims 1 to 6, wherein the one or more processors are further configured to route processed data from the first and second DDTs (214; 214a, 214b) to a queue (306a-306d) configured to feed an associated one of a report generator (308) or a file generator (310a-310c), wherein the queue (306a-306d) is configured to temporarily store the processed data for the associated one of the report generator (308) or the file generator (310a-310c).

8. The system of any one of claims 1 to 7, wherein the one or more processors are further configured to:
generate an electronic health record (EHR) based on processed data output by the first and second DDTs (214; 214a, 214b); and
output the EHR.

9. The system of any one of claims 1 to 8, further comprising a third DDT (214; 214c) configured to process data from a third type of medical device (124; 124c) that is different from the first and second types of medical devices (124; 124a, 124b).

10. The system of any one of claims 1 to 8, further comprising a third DDT (214; 214c) configured to process data from the first type of medical device (124; 124a).

11. The system of any one of claims 1 to 10, further comprising a transmitter, wherein
the one or more processors are configured to generate a report or a file based on processed data output by at least one of the first and second DDTs (214; 214a, 214b);
the transmitter is configured to transmit the report or file to i) a predetermined electronic address associated a medical device (124) that is associated with the processed data, ii) a location associated with a type of data in the report or file, or iii) a location associated with a type of medical device (124) that is associated with the processed data.

12. The system of any one of claims 1 to 11, further comprising a second memory configured to store processed patient data and electronic health records, wherein the second memory is configured to be accessed by a clinician application (110).

13. A computer implemented method, comprising:
utilizing one or more processors that, when executing specific program instructions, perform:
receiving patient data, the patient data comprising physiological data and at least one corresponding unique device identifier (ID) associated with a first medical device (124);
determining whether the unique ID is associated with a first DDT (214; 214a) or a second DDT (214; 214b), wherein the first DDT (214; 214a) is configured to process data from a first type of medical device (124; 124a) and the second DDT (214; 214b) is configured to process data from a second type of medical device (124; 124b) that is different from the first type of medical device (124; 124a); and
routing patient data associated with the first medical device (124) to the first DDT (214; 214a) or the second DDT (214; 24b) that is associated with the unique ID.

14. The method of claim 13, further comprising:
responsive to receiving the patient data, outputting processed data with the first DDT (214; 214a); and
routing the processed data to a business service (334) configured to output at least one of a file or a report.

15. The method of claim 13 or 14, further comprising:
responsive to the DDTs (214; 214a, 214b) outputting processed data, storing the processed data in a memory;
processing at least a portion of the processed data in the memory based on at least one of artificial intelligence, machine learning, and data analytics; and
responsive to storing the further processed data in the memory:
i) output, to a location remote from the one or more processors, recommendations for setting changes associated with one of the first or second medical devices (124),
ii) output, to a location remote from the one or more processors, instructions to implement programmed settings associated with one of the first or second medical devices (124),
iii) output, to a location remote from the one or more processors, instructions to automatically remotely program one of the first or second medical devices (124), and/or
iv) output, to a location remote from the one or more processors, an alert.
